# EUROPEAN PATENT APPLICATION

(11) **EP 4 847 030 A1**
(43) Date of publication of application: **02.12.2026**
(21) Application number: 25382535.0
(22) Date of filing: 27.05.2025
(51) Int. Cl.: A61K 35/76, A61K 38/16, A61P 31/04, C07K 14/195, C12N 7/00

(54) **PHAGE TAIL-LIKE BACTERIOCIN-TAILOCINS (R- AND F-TYPES) AS ADJUVANTS FOR LYTIC PHAGES**

(71) Applicant: Fundación Pública Galega de Investigación Biomédica INIBIC, 15006 A Coruña (ES); Servizo Galego De Saúde, 15703 Santiago de Compostela, A Coruna (ES)
(72) Inventor: TOMAS CARMONA, María del Mar, 15006 A Coruña (ES); BLASCO OTERO, Lucía, 15006 A Coruña (ES); IBARGUREN QUILES, Clara, 15006 A Coruña (ES); BLERIOT RIAL, Inés María, 15006 A Coruña (ES)
(74) Representative: Hoffmann Eitle

(57) **Abstract**

The present invention relates generally to antimicrobial therapies, specifically to the medical use of bacterially derived antimicrobial agents. More particularly, the invention concerns the therapeutic application of phage tail-like bacteriocins (PTLBs), preferably tailocins, in combination with bacteriophages for the targeted treatment and prevention of bacterial infections, including multidrug-resistant bacterial strains, in humans and animals.

## Description

### TECHNICAL FIELD

The present invention relates generally to antimicrobial therapies, specifically to the medical use of bacterially derived antimicrobial agents. More particularly, the invention concerns the therapeutic application of phage tail-like bacteriocins (PTLBs), or tailocins, in combination with bacteriophages for the targeted treatment and prevention of bacterial infections, including multidrug-resistant bacterial strains, in humans and animals.

### BACKGROUND ART

Antimicrobial resistance (AMR) presents a significant global health threat, rendering numerous conventional antibiotics increasingly ineffective against pathogenic bacteria. The rise of multidrug-resistant (MDR) bacteria, including those categorized as ESKAPE pathogens *(Enterococcus faecium, Staphylococcus aureus, Klebsiella pneumoniae, Acinetobacter baumannii, Pseudomonas aeruginosa,* and *Enterobacter* species), underscores the urgent need for novel antimicrobial strategies. While traditional antibiotics frequently target essential metabolic pathways common to many bacterial species, this broad-spectrum approach has significantly contributed to the development of resistance and disruption of beneficial microbiota.

Bacteriophages and their derivatives, such as tailocins, have emerged as promising alternatives due to their narrow spectrum of activity, specificity toward target bacteria, reduced likelihood of resistance development, and minimal collateral damage to commensal microbiomes. Tailocins are structurally similar to bacteriophage tails but are encoded by bacterial genomes, functioning as precise antibacterial agents capable of selective binding and lethally puncturing target bacterial cells.

Prior research has demonstrated the effectiveness of tailocins against various pathogenic bacteria; however, clinical translation and detailed therapeutic protocols have yet to be fully developed or standardized. Thus, there remains a significant unmet need for optimized, clinically validated tailocins-based therapies capable of effectively addressing bacterial infections while minimizing resistance selection pressures and maintaining microbiome integrity.

The present invention addresses this gap by providing novel methods, compositions, and therapeutic protocols utilizing tailocins complex and bacteriophage, thus offering a targeted and innovative solution for combating antibiotic-resistant bacterial infections.

### BRIEF DESCRIPTION OF THE FIGURES

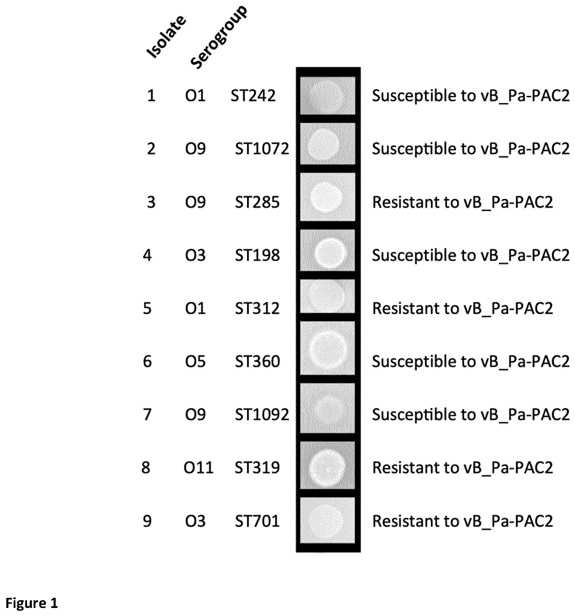

**Figure 1****.** Tailocins **complex host range.** Positive results of the host range assay done with the tailocins complex on nine different isolates as it is observed a lysed halo without bacterial growth. Four out of nine of these isolates are also susceptible to the phage vB_Pa-PAC2.

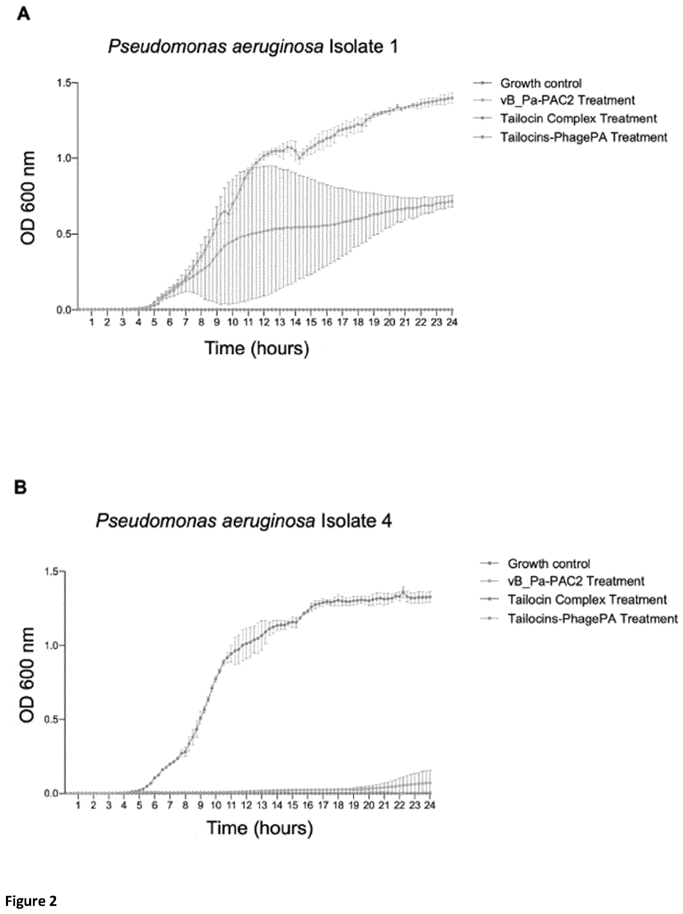

**Figure 2****. Time killing curve assay.** Results of killing curves of *P. aeruginosa* isolate 1 (A) and isolate 4 (B) without treatment and with the different treatments: vB_Pa-PAC2 phage and tailocins complex at 24 h. The bacterial growth is reduced when treated with the phage vB_Pa-PAC2 (yellow) in comparison with the control of growth (red). Additionally, no growth is observed when treated with the tailocins complex (blue) or the Tailocins-PhagePA (green).

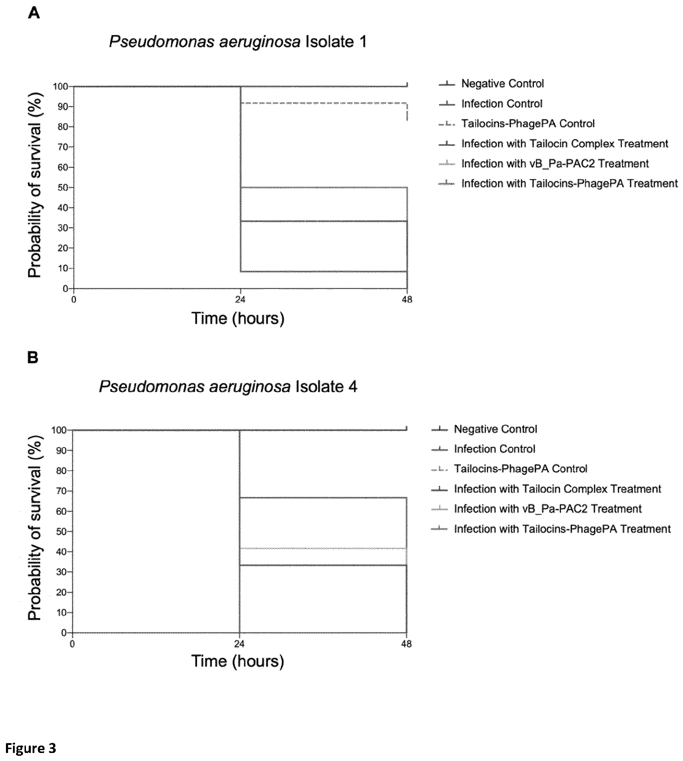

**Figure 3****. Survival curves of *G. mellonella* infection model.** (A) *G. mellonella* survival when infected with the *P. aeruginosa* Isolate 1. When treated with the tailocins complex (blue) the survival is increased in comparison with the infection control (red) or the phage vB_Pa-PAC2 treatment (yellow) at 24 h. More survival is observed when the infection is treated with the Tailocins-PhagePA (green) also at 24 h. (B) *G. mellonella* survival when infected with the *P*. *aeruginosa* Isolate 4. A slightly increase survival was observed when the larvae was treated with tailocins complex (blue) or phage vB_Pa-PAC2 (yellow). However, the survival is significant improved with the Tailocins-PhagePA (green).

### BRIEF DESCRIPTION OF THE INVENTION

The present invention relates to a composition (from hereinafter referred to as the bacteriophage-PTLB complex composition) comprising at least one bacteriophage, or a nucleic acid encoding for the same, at least one phage tail-like bacteriocin (PTLB), or a tailocin or tailocins-complex, or a nucleic acid encoding for the same, and, optionally, a pharmaceutically or veterinary acceptable excipient; wherein the at least one bacteriophage and the at least one phage tail-like bacteriocin (PTLB), or a tailocin or tailocins-complex, are both specific against the same particular bacterial target species, or at least against bacterial strains within the same particular target species.

In a further embodiment, the bacteriophage-PTLB complex composition would comprises at least two or more bacteriophages, or nucleic acids encoding for the same, having lytic activity against one or more target species, and at least one phage tail-like bacteriocin (PTLB), or a tailocin or tailocins-complex, specific against the same target species of at least one of the bacteriophages present in the composition, or at least against bacterial strains within the same particular target species. Preferably, the target species is *P. aeruginosa,* and more specifically an antibiotic-resistant *P. aeruginosa* strain. In a preferred embodiment, the specific bacteriophage against *P. aeruginosa* is the *Pseudomonas aeruginosa* phage vB_Pa-PAC2 (NCBI txid: 3127731), and the specific tailocin or tailocins-complex is the tailocins-complex of SEQ ID NO 1 or any functional variant thereof.

In another preferred embodiment, the composition would be a liquid, semi-liquid, solid or lyophilized formulation.

In yet another preferred embodiment, the bacteriophage-PTLB complex composition comprises between 100 and 10,000 Lethal Killing Units (LKU) of the phage tail-like bacteriocin (PTLB), or a tailocin or tailocins-complex. Furthermore, in another preferred embodiment, the composition would comprise a bacteriophage concentration between 10⁴ and 10¹² plate forming units per mL (PFU/mL) of each bacteriophage.

In yet another preferred embodiment, the bacteriophage-PTLB complex composition is for use in the treatment of an infection in a mammal.

In yet another preferred embodiment, the bacteriophage-PTLB complex composition is for use in the treatment of an infection of the respiratory tract.

In yet another preferred embodiment, the bacteriophage-PTLB complex composition is for use in improving the condition of a mammal by modifying the microbial flora in said mammal.

In yet another preferred embodiment, the bacteriophage-PTLB complex composition is use, in vitro or ex-corpus, for decontaminating a material.

A further aspect of the invention relates to a composition comprising at least one phage tail-like bacteriocin (PTLB), or a tailocin or tailocins-complex, for use in a method of treating an infection of a target species in a subject in need thereof, wherein the at least one phage tail-like bacteriocin (PTLB), or a tailocin or tailocins-complex, is administered to the subject prior to, simultaneously or subsequently to a composition comprising at least one bacteriophage; and wherein the at least one bacteriophage and the at least one phage tail-like bacteriocin (PTLB), or a tailocin or tailocins-complex, are both specific against the same target species, or at least against bacterial strains within the same particular target species. Preferably, the composition comprising at least one phage tail-like bacteriocin (PTLB), o a tailrocin or tailocins-complex, and the composition comprising at least one bacteriophage can be jointly present as a single composition or as separate compositions. Ideally, the target species is selected from any one of the bacterial pathogens selected from the list consisting of: *Pseudomonas aeruginosa, Escherichia coli, Klebsiella pneumoniae, Acinetobacter baumannii, Salmonella enterica, Shigella flexneri, Enterobacter cloacae, Proteus mirabilis, Staphylococcus aureus, Staphylococcus epidermidis, Streptococcus pneumoniae, Streptococcus pyogenes, Streptococcus agalactiae, Clostridium difficile, Enterococcus faecalis, Enterococcus faecium, Bacillus cereus, Listeria monocytogenes, Vibrio cholerae, Vibrio parahaemolyticus, Campylobacter jejuni, Helicobacter pylori, Burkholderia cepacia, Burkholderia pseudomallei, Neisseria meningitidis, Neisseria gonorrhoeae, Haemophilus influenzae, Yersinia enterocolitica, Yersinia pestis,* and related bacterial species possessing compatible surface receptors or molecular markers necessary for binding, inhibition, or lysis by phage tail-like bacteriocin (PTLB), or a tailocin or tailocins-complex, and bacteriophages, as confirmed by standard microbiological assays. More preferably, at least one bacteriophage (*Pseudomonas* phage vB_Pa-PAC2 (NCBI:txid3127731)) and at least one tailocins complex (SEQ ID NO 1) or any functional variant thereof, should target *Pseudomonas aeruginosa.*

Finally, the present invention also encompasses any composition comprising, consisting essentially of or consisting of SEQ ID NO 1 or any functional variant thereof.

### DESCRIPTION OF THE EMBODIMENTS OF THE INVENTION

The present invention, as illustrated in figure 2, shows a time killing curve assay performed with the seed of bacteriophage, vB_Pa-PAC2, which action against infection of isolates 1 and 4 did not eradicate completely the growth of bacteria (OD₆₀₀ₙₘ= 0.716 and 0.0712, respectively). In the case of the tailocin complex it can be observed that it eliminates completely the infection (OD₆₀₀ₙₘ= 0.0030 and 0.0036, respectively). Finally, with the Tailocins-PhagePA it can be seen an improvement in the eradication of the bacterial infection (OD₆₀₀ₙₘ= 0.0011 and 0.0014, respectively (Figure 2).

On the other hand, the larval survival assay illustrated in figure 3 showed that the Tailocins-PhagePA was not toxic for the *G. mellonella.* Moreover, this figure further shows that in the case of the group of larvae infected with the isolate 1 and treated with the bacteriophage did not improve the survival at 24 h, since there is an 8.33 % of survival, same as the group of untreated larvae. On the other hand, treatment of this infection with the tailocin complex slightly improves survival by 33.33 %. A large increase (66.67 %) in survival of *G. mellonella* was observed in the group treated with the combination of both treatments (Tailocins-PhagePA) at 24 h (Figure 3A). Moreover, when the larvae were infected with the isolate 4 and treated with the phage alone or with tailoicins complex alone, it is observed a slight improvement in the survival (33.33 % and 41.67 %, respectively). However, it can be seen that the treatment with the Tailocins-PhagePA, again, significantly increases the percentage of larvae survival up to 66.67 % (Figure 3B).

The results obtained suggest the great potential of **Tailocins complexes (R- and F-types)** as adjuvants to phage therapy. The evidence of improvement of infection in both *in vitro* and *in vivo* studies is promising for this therapeutic field, supported also by the low toxicity resulting from the use of the product.

The present invention is thus based on the identification of phage tail-like bacteriocins (PTLBs), preferably a tailocin or tailocins-complex, as adjuvants to phage therapy, in particular as adjuvants to therapy with phages. Also provided for such purpose are modified or non-natural phage tail-like bacteriocins (PTLB), preferably a tailocin or tailocins-complex, such as those that have been engineered to have altered bactericidal specificity. Accordingly, provided herein are nucleic acid molecules encoding natural or non-natural phage tail-like bacteriocins (PTLBs), preferably a tailocin or tailocins-complex, integration vector constructs containing such nucleic acids operably linked to a heterologous promoter, producer cells that do not naturally produce phage tail-like bacteriocins (PTLBs), preferably a tailocin or tailocins-complex, but containing such nucleic acid molecules or vectors, as well as the encoded phage tail-like bacteriocins (PTLBs), preferably a tailocin or tailocins-complex.

To facilitate understanding of the invention, a number of terms are defined below.

As used herein, the term "bacteriophage" or "phage" refers to a virus that infect bacteria. A functional phage particle comprising a nucleic acid genome packaged in a proteinaceous envelope or capsid. The term also refers to portions of the bacteriophage, including, e.g., a head portion, or an assembly of phage components, which provide substantially the same functional activity.

As used in this document, the term phage tail-like bacteriocin (PTLB), preferably a tailocin or tailocins-complex, refers to multiprotein structures similar to a phage tail that may include, F-type bacteriocins (FTBs) and R-type bacteriocins (RTBs), in reference to its flexible or rigig morphology. They are genetically encoded in a cluster in the bacterial genome.. For example, a monocin is a tailocin. A tailocin may be natural or non-natural, that is it exists in nature or does not exist in nature, respectively.

The term "tailocins-complex" refers to a tailocin complex composed of an R-type and an F-type tailocin, which are encoded in the same cluster.

The term "phenotypic characteristic" designates more preferably the morphology and/or host-range of a bacteriophage. Methods for phenotyping bacteriophages are well known per se in the part and include, for example, determining bacterial host range and/or activity against the biofilm produced by certain bacterial strains.

The term "lytic activity" as used in the invention designates the property of a bacteriophage to cause lysis of a bacterial cell. The lytic activity of a bacteriophage can be tested on *P. aeruginosa* strains according to techniques known.

The term "variant" of a reference bacteriophage designates a bacteriophage that exhibis variation(s) in the genomic sequence and/or encoded polypeptide(s) compared to the reference bacteriophage, while retaining the same phenotypic characteristic as the reference bacteriophage. Variants typically comprise e.g., silent mutations, conservative mutations, minor deletions, and/or minor replications of genetic material, and retain phenotypic characteristics of the reference bacteriophage. In a preferred embodiment, variants according to the invention retain any observable characteristic or property that is dependent upon the genome of the bacteriophage of the invention, i.e. phenotypic characteristics of said bacteriophage and/or lytic activity against the *P. aeruginosa* strains. Preferred variants have less than 5% nucleic acid variation as compared to the genome of the reference bacteriophage, even more preferably less than 4%, more preferably less than 2%. Alternatively, or in combination, variants have preferably less than 5% amino acid variation in a coded polypeptide sequence as compared to a polypeptide of the reference bacteriophage.

The term "specific" or "specificity" in relation to a bacteriophage or to tailocins refers to the type of host that said bacteriophage is able to infect or lysed. A bacteriophage "specific" for *P*. *aeruginosa* more preferably designates a bacteriophage which can infect one or several *P*. *aeruginosa* strains and which cannot infect non-P.aeruginosa bacteria under physiological conditions. As used herein, the term "polypeptide" refers to polypeptides of any size, including small peptides of e.g., from 5 to 20 amino acids, longer polypeptides, proteins or fragments thereof.

The term "treatment" or "therapy" designates a curative or a prophylactic treatment of a disease. A curative treatment is defined as a treatment that results in a cure of a disease, or a treatment that alleviates, reduces, stabilizes, or eliminates the symptoms of a disease or the suffering that it causes, directly or indirectly, or that improves a subject condition or reduces progression of a disease. A prophylactic treatment comprises a treatment resulting in the prevention of a disease, and/or a treatment reducing and/or delaying the incidence of a disease or the risk of its occurrence.

The term "biofilm" as used herein designates a heterogeneous bacterial formation growing on various surfaces; preferably a bacterial community growing embedded in an exopolysaccharide matrix adhered onto solid biological or non-biological surfaces.

The term "compromise" as used herein refers to any alteration of the integrity. By compromising a bacterial biofilm, it is understood a penetration of the biofilm by bacteriophage, an infection of bio film-associated bacteria and/or a lysis thereof and/or a partial or an entire clearing of the biofilm (i.e., by stopping colonization and/or disrupting biofilms).

The term "sample", as used herein, means any sample containing cells. Examples of such samples include body fluids such as blood, plasma, saliva, or urine, as well as biopsies, organs, tissues or cell samples. The sample may be treated prior to its use.

As used herein, the term "subject" or "patient" refers to an animal, preferably a mammal, even more preferably a human, including adult and child. The term "subject" also encompasses non-human animals, in particular non-human mammals such as pets (e.g., dogs, cats), horses, cows, goats, pigs, sheep and non-human primates, among others.

The term "efficacy" of treatment or "response" to a bacteriophage therapy as used herein refers to a treatment which results in a decrease in the number of bacterial concentrations, such as *P*. *aeruginosa,* strains in a subject after bacteriophage treatment when compared to the number of strains before treatment. A "good responder" subject refers to a subject who shows or will show a clinically significant recovery when treated with a bacteriophage therapy.

The term "Cocktail or PTMPs" of bacteriophages designates a combination of different types of bacteriophages. The bacteriophages in a PTMPs are preferably formulates together in a same vessel or packaging, although they may be used as kits of parts wherein some of the bacteriophages are formulated or packaged separately and combined when used or administered.

The bacteriophage of the invention can be prepared by standard culture, isolation and purification methods. For example, *P. aeruginosa* are cultured and infected by a sample of a seed of bacteriophage, and then treated to remove bacterial cells and debris. The enriched bacteriophage solution can be plated in a medium, for example TA agar medium, with embedded susceptible host strains of *P. aeruginosa* to obtain plaques of lysis. Then, single plaque can be picked out for subsequent bacteriophage purification and amplification. One or more cycles of selective adaptation of bacteriophages of the invention may be performed, for example by mixing bacteriophages with the competent strain of *P. aeruginosa,* followed by addition of a growth medium and incubation at selected test growing conditions. Following centrifugation, the cleared amplified supernatant is filtered through filter (0.22 or 0.45 µm) and subjected to another cycle of selective adaptation or tested for presence of lytic activity. The titer of phage in a suspension and the visualization of plaque morphology of bacteriophages of the invention may then be assessed by known methods, for example by plaque counting. Additionally, processing bacteriophage of the invention in various forms (liquid, lyophilized, etc.) for short-, long-, freeze- or any other kind of storage can be carried out by any suitable method as it is well-known in the art (see e.g., Clark, 1962).

The activity of the bacteriophages of the invention can be assessed by methods well- known in the art, such as plaque assay also known as double agar method, based on the growing of host bacteria and followed by the addition of a bacteriophage by assessing their ability to kill the host bacterial cell. In the plaque assay method, the bacteriophage induces lysis of target *P*. *aeruginosa* strains after a period of incubation in soft agar medium, resulting in zones of clearing on the plate known as plaques.

Therefore, a first aspect of the invention relates to compositions, (also herein referred to as bacteriophage-PTLB complex compositions), comprising at least one bacteriophage, more preferably at least 2 or more and, optionally, a pharmaceutically or veterinary acceptable excipient, and at least one phage tail-like bacteriocin (PTLB), or a tailocin or tailocins-complex, wherein at least one bacteriophage and the at least one phage tail-like bacteriocin (PTLB), or a tailocin or tailocins-complex, are both specific against the same particular target species, or at least against the same or closely related bacterial strains within the same particular target species.

As used herein, the term "target species" refers specifically to the bacterial species, strain, or closely related group of bacteria against which the phage tail-like bacteriocin (PTLB), or a tailocin or tailocins-complex, and the bacteriophage exhibits selective antibacterial activity, as evidenced by its ability to bind, inhibit growth, or cause lysis. The term encompasses all bacterial isolates sharing the specific surface receptors or molecular markers recognized by the antibacterial agent, as confirmed by standard microbiological assays.

It is noted that, in a preferred embodiment of the first aspect of the invention, combinations of bacteriophages and/or phage tail-like bacteriocins (PTLBs), or a tailocin or tailocins-complex, may be used to expand the host spectrum and produce still higher effective antibacterial compositions. More particularly, the invention relates to an antibacterial composition comprising at least two bacteriophages having lytic activity against a target species, wherein the antibacterial composition further comprises at least one phage tail-like bacteriocin (PTLB), or a tailocin or tailocins-complex, specific against the same particular target species of at least one of the bacteriophages present in the composition, or at least against closely related bacterial strains within the same particular target species.

In a preferred embodiment, the compositions of the invention comprise at least three, even more preferably at least four distinct bacteriophages, as long as the composition further comprises at least one phage tail-like bacteriocin (PTLB), or a tailocin or tailocins-complex, specific against the same particular target species of at least one of the bacteriophages present in the composition, or at least against closely related bacterial strains within the same particular target species.

Particular compositions of the invention comprise at least the bacteriophage named *Pseudomonas* phage vB_Pa-PAC2 (NCBI:txid3127731) and the tailocins complex of SEQ ID NO 1 or any functional variant thereof. As used herein, the term "functional variant" of tailocin refers to any tailocins complex derivative, mutant, fragment, analog, or homolog thereof, which retains substantially equivalent antibacterial activity and specificity against the defined target species. Such variants may differ from the naturally occurring or originally described tailocins complex by one or more amino acid substitutions, deletions, insertions, modifications, or combinations thereof, provided that the biological function-specifically the ability to recognize, bind, and lyse or inhibit growth of the targeted bacterial species-is maintained, as assessed by standard microbiological assays.

The compositions of the invention may further comprise additional antibacterial agents, particularly other bacteriophages having distinct host specificity.

The compositions of the invention may comprise any effective amount of the selected bacteriophage(s) or phage tail-like bacteriocin (PTLB), or a tailocin or tailocins-complex.

The antibacterial compositions of the invention may be in various forms, such as liquid, semi-liquid, solid or lyophilized formulations. The compositions of the invention preferably comprise a suitable diluent or carrier, such as a pharmaceutically or veterinary acceptable excipient or carrier. Compositions according to the present invention may include any excipient or carrier, such as thickeners, diluents, buffers, preservatives, surface active agents and the like, in addition to the bacteriophage(s) of choice. Such includes physiologically acceptable solutions or vehicles that are harmless or do not cause any significant specific or non-specific immune reaction to an organism or do not abrogate the biological activity of the bacteriophage. For liquid formulation, saline, sterile water, Ringer's solution, buffered physiological saline, albumin infusion solution, dextrose solution, maltodextrin solution, glycerol, ethanol, and mixtures thereof may be used as a pharmaceutically or veterinary acceptable excipient or carrier. If appropriate, other conventional additives such as thickeners, diluents, buffers, preservatives, surface active agents, antioxidants and bacteriostatic agents may be added. Further, diluents, dispersants, surfactants, binders and lubricants may be additionally added to the composition to prepare injectable formulations such as aqueous solutions, suspensions, and emulsions, oral formulations such as pills, capsules, granules, or tablets, or powdered formulations. Formulations for topical administration may include, band aids, dressings, patches, films, ointments, lotions, creams, gels, drops, suppositories, sprays, tampons, sanitary towels, liquids and powders. Formulations for decontamination or for medical use may also include aerosols or sprays.

The compositions of the invention may be used in the medical field, including the human or veterinary medical areas, for e.g. the treatment of an infection in a mammal or for improving a subject's condition. The compositions may be used to kill any target species in an organism, or at any closely related bacterial strains within the same particular target species, for treating an infection. The composition may also be used for improving the condition of a mammal by modifying the microbial flora in said mammal. In particular, the compositions of the invention can specifically remove *P. aeruginosa* strains on the skin or mucous membranes of a mammal, thus modifying its microbial flora and restoring a proper balance.

In a particular embodiment, the invention also relates to a method for treating an infection in a mammal comprising the administration to said mammal of a composition comprising at least a bacteriophage and at least a phage tail-like bacteriocin (PTLB), or a tailocin or tailocins-complex, or nucleic acids and/or polypeptides as defined above.

The invention also relates to the use of a composition, bacteriophage, phage tail-like bacteriocin (PTLB), or a tailocin or tailocins-complex, nucleic acid or polypeptide as described, for the manufacture of a medicament for treating an infection in a mammal, or for restoring microbial flora in said mammal. The compositions of the invention may be used to treat any target species against which the disclosed phage tail-like bacteriocin (PTLB), or a tailocin or tailocins-complex, and bacteriophage exhibit selective antibacterial activity. Target species include, but are not limited to, the following bacterial pathogens: *Pseudomonas aeruginosa, Escherichia coli, Klebsiella pneumoniae, Acinetobacter baumannii, Salmonella enterica, Shigella flexneri, Enterobacter cloacae, Proteus mirabilis, Staphylococcus aureus, Staphylococcus epidermidis, Streptococcus pneumoniae, Streptococcus pyogenes, Streptococcus agalactiae, Clostridium difficile, Enterococcus faecalis, Enterococcus faecium, Bacillus cereus, Listeria monocytogenes, Vibrio cholerae, Vibrio parahaemolyticus, Campylobacter jejuni, Helicobacter pylori, Burkholderia cepacia, Burkholderia pseudomallei, Neisseria meningitidis, Neisseria gonorrhoeae, Haemophilus influenzae, Yersinia enterocolitica, Yersinia pestis,* and related bacterial species possessing compatible surface receptors or molecular markers necessary for binding, inhibition, or lysis by tailocins complex and bacteriophages, as confirmed by standard microbiological assays.

In particular, the compositions of the invention may be used to treat various *P. aeruginosa-*mediated infections, particularly of the respiratory system. *P. aeruginosa* is one of the major microbiological agents responsible for the pathology, especially in the young children and elderly populations, as well as, in immunocompromised, cystic fibrosis, high burn and poly-traumatized patients. From a conservative stand point, about 20 % of these germs are resistant to several or all the antibiotics from our therapeutic arsenal (remarkably, the highest number or resistant cases is being observed in intensive care unit. As a consequence, estimated figures indicate that at least 90 000 pneumonia cases in USA an 120 000 in Europe are induced by antibiotic multi-resistant *P. aeruginosa* bacterial strains. The invention is thus particularly suited for treating pneumonia associated with, or caused by, *P. aeruginosa* infection. An object of the invention thus resides in a method of treating pneumonia in a subject in need thereof, comprising administering a composition of the invention to said subject. The method is particularly suited for treating pneumonia induced by antibiotic-resistant *P. aeruginosa* bacteria. The subject may be any human subject, such as children, adults or elderly people.

The compositions of the invention may be administered by any convenient route, including intravenous, oral, transdermal, subcutaneous, mucosal, intramuscular, intrapulmonary, intranasal, parenteral, rectal, vaginal and topical. In an ideal embodiment, the compositions are administered by using the intrapulmonary or intranasal route. The compositions may be administered directly or indirectly, e.g., via a support. In this regard, the compositions may, for example, be applied or sprayed to the afflicted area. Compositions of the invention can also be administered by oral or parenteral routes. The dosage suitable for applying, spraying, or administrating the compositions of the present invention can be adjusted by the skilled person depending on a variety of factors including formulation, mode of administration, age, weight, sex, condition, diet of the mammal being treated at the time of administration, route of administration, and reaction sensitivity. A physician having ordinary skills in the art can readily determine and prescribe the effective amount of the composition required.

The dosing can also be adjusted by the skilled person so that a lytic activity against the target species is obtained. Also, the compositions of the invention are strictly unable to affect mammalian cells, and are therefore specific and devoid of side effects *in vivo.* The invention also relates to the use of a composition of the invention for decontaminating a material. Due to their potent antibacterial effect, and to their ability to even compromise the integrity of a bacterial biofilm, the compositions of the invention can be used as decontaminating agent, to eliminate or at least cause a reduction in bacterial numbers on a material. Such methods may be applied for the treatment of a variety of biological or non-biological surfaces in both medical and non-medical contexts, including solid materials or devices such as, for example, contact lenses, surfaces of devices to be implanted into the body, pipes, ducts, laboratory vessels, textiles, etc.

The invention also relates to methods for treating an infection of a target species in a subject by administering to said subject an amount of at least one bacteriophage, and an amount of at least one phage tail-like bacteriocin (PTLB), or a tailocin or tailocins-complex, wherein the at least both are specific against the same target species or against closely related bacterial strains within the same particular target species.

The invention further relates to methods of treating an infection of a target species in a subject by administering to said subject an amount of at least one bacteriophage, and an amount of at least one phage tail-like bacteriocin (PTLB), or a tailocin or tailocins-complex, wherein both are specific against the same target species or against closely related bacterial strains within the same particular target species. The application of both elements should be able to be present in a single composition or in a separate composition where they can be administered simultaneously or in deferred time according to the requirement of the subject. In one embodiment, the infection by the target species include, but are not limited to, the following bacterial pathogens: *Pseudomonas aeruginosa, Escherichia coli, Klebsiella pneumoniae, Acinetobacter baumannii, Salmonella enterica, Shigella flexneri, Enterobacter cloacae, Proteus mirabilis, Staphylococcus aureus, Staphylococcus epidermidis, Streptococcus pneumoniae, Streptococcus pyogenes, Streptococcus agalactiae, Clostridium difficile, Enterococcus faecalis, Enterococcus faecium, Bacillus cereus, Listeria monocytogenes, Vibrio cholerae, Vibrio parahaemolyticus, Campylobacter jejuni, Helicobacter pylori, Burkholderia cepacia, Burkholderia pseudomallei, Neisseria meningitidis, Neisseria gonorrhoeae, Haemophilus influenzae, Yersinia enterocolitica, Yersinia pestis,* and related bacterial species possessing compatible surface receptors or molecular markers necessary for binding, inhibition, or lysis by bacteriophages and/or tailocins complex, as confirmed by standard microbiological assays.

As described herein, methods of treating an infection of a target species in a subject by administering an amount of at least one bacteriophage, and at least one phage tail-like bacteriocin (PTLB), or a tailocin or tailocins-complex, can be used to inhibit growth, survival, or replication of the target species. In some embodiments, the subject in need to be treated is a human child or fetus or other young animal which has yet to reach maturity. *Pseudomonas aeruginosa* bacteriophage vB_Pa-PAC2 (NCBI:txid3127731) and tailocin complex (SEQ ID NO 1) or any functional variant thereof can be used to perform such treatment.

The term "comprising", which is used interchangeably with "including," "containing," or "characterized by," is inclusive or open-ended language and does not exclude additional, unrecited elements or method steps. The phrase "consisting of" excludes any element, step, or ingredient not specified in the claim. The phrase "consisting essentially of" limits the scope of a claim to the specified materials or steps and those that do not materially affect the basic and novel characteristics of the claimed invention. The present disclosure contemplates embodiments of the invention compositions and methods corresponding to the scope of each of these phrases. Thus, a composition or method comprising recited elements or steps contemplates particular embodiments in which the composition or method consists essentially of or consists of those elements or steps.

The following examples are merely for illustration purposes only.

### EXAMPLES

### METHODS AND MATERIALS

### BACTERIAL, PHAGE AND CULTURE CONDITIONS

The *Pseudomonas aeruginosa* isolates used in this work were grown in Luria-Bertani (LB) broth medium (0.5% yeast extract; 0.5% NaCl; 1% tryptone) at 37 °C and shaking conditions (180 rpm). When necessary, LB broth medium was supplemented with 1.5% agar to obtain solid medium in plates. Serogroup of these isolates was based on the O-specific antigen (OSA) gene cluster sequence. It was determined using the bioinformatic tool PAst (1.0) (1).

The characterization of the tailocin complex was done by the double agar method that consists of a base with TA medium (0.5% Na Cl, 1 % Tryptone and 1.5 % agar) and on top a suspension of soft agar medium (0.5 % NaCl, 1 % Tryptone and 0.3 % agar).

In this invention the phage vB_Pa-PAC2 isolated from the sewage water from the Hospital Materno-Infantil Terresa Herrera was used. Its sequence was deposited in the NCBI (nucleotide sequence: PP408249.1) included in the bioproject PRJNA1082106.

### PHAGE PROPAGATION

The seed of phage vB_Pa-PAC2 was amplified in TA liquid media supplemented with 1 mM CaCl₂, with shaking (180 rpm) at 37 °C, by infecting an early logarithmic growth phase *P. aeruginosa* culture (OD₆₀₀ₙᵣₙ = 0.3-0.4). After lysis, i.e., when the culture appeared clear, bacteria and debris were removed by centrifugation (4350 × g 10 min) and filtration (0.45 µm). Finally, the supernatants were titrated by the double-layer method by serial dilution in SM buffer (10 % NacL (1 M), 1 % MgSO₄ (1M), 2 % Tris-HCL (1 M)) and stored at 4 °C.

### TAILOCIN BIOINFORMATICS ANALYSIS

### - Identification and annotation of tailocin complex cluster in silico

The genome sequence of the *P. aeruginosa* isolate 10 used in this invention was annotated by several bioinformatic tools, so that it could be searched the word "pyocin", in order to find the genomic region of tailocins complex cluster. The tailocin complex sequence was located by the identification of tailocins complex flanking genes: the *trpD* and *trpE.* Once the PTLB complex sequence was stablished it was annotated by HMMER (version v3.4) (2), which predicts protein functions.

### - Tailocin complex cluster type identification

The cluster of the tailocin complex was compared by protein sequence homology with Blastp (https ://blast.ncbi.nlm.nih.gov/Blast.cgi?PROGRAM=blastp&PAGE TYPE=BlastSearch&BLAST SPEC=&LINK LOC=blasttab&LAST PAGE=blastn) (3).For this comparison, the reference protein used for each subtype were: R1 (ARI05994.1), R2 (AAG04009.1), R3 (ABP93392.1), R4 (ABP93394.1), R5 (ABP93396.1), F2 (AAG04035.1) and F(PA14) (ABJ15607.1). Additionally, for the F-type tailocincomplex subtypes classification, the reference sequences of the last six proteins located in the C-terminal of the cluster, described and published by Saha *et al.*(4).

### TAILOCINS COMPLEX EXTRACTION, CONCENTRATION, HOST RANGE AND QUANTIFICATION

### - Extraction and concentration

The extraction, purification, and concentration of the tailocin complex was performed with a previous adapted method (5). Briefly, an overnight culture of the selected isolate was diluted in LB broth medium (1:100) and incubated at 37°C in shaking conditions (180 rpm). When the culture reached an optical density of 0.3-0.4 (OD₆₀₀ₙₘ), 10 µg/ml of mitomycin C (Sigma-Aldrich) were added and incubated until the culture was cleared. To ensure maximum bacterial lysis, the lysed culture was treated with 1% chloroform for 20 min at room temperature and centrifuged at 3700 rpm for 10 min. The supernatant containing the tailocin complex was filtered through a 0.45 µm filter. For concentration, the supernatant containing the tailocin complex was precipitated overnight at 4 °C with 10% PEG (polyethylene glycol 6000; Sigma-Aldrich) and 0.5 M NaCl. The next day the tailocin complex was collected by centrifugation for 15 min at 11000 rpm and 4 °C. The supernatant was discarded, and the pellet was resuspended in SM buffer, and equal volume of chloroform was added to the final volume (1:1) and incubated with gentle shaking for 20 min and then centrifuged for 10 min at 4000 rpm. The aqueous phase was recovered and incubated with 10 µg/mL Proteinase K at 56 ºC for 1 h, in order to degrade putative prophages induced by mitomycin C. Finally, the tailocin complex was purified and dialyzed with a 100 KDa Amicon^{®} Ultra-15 (Merck Milipore) system. The tailocin complex solution obtained was stored at 4 °C until use.

### - Host Range

The host range or killing activity was tested by the spot test technique through the double agar method (6), with the *P. aeruginosa* isolates tested, previously grown until reaching an optical density of 0.6 (OD₆₀₀ₙᵣₙ). Once the top layer of soft TA has solidified, a 5 µL drop of different dilutions of the suspension containing the tailocin complex were deposited. Finally, the plates were incubated overnight at 37 °C. After incubation, the plates were evaluated, marking as positive those clear spots without bacterial growth. It was considered negative those turbid spots with bacterial growth.

### - Quantification

The killing activity of the tailocin complex was indirectly quantified by determining the lethal killing units (LKU) using Poisson's distribution, as previously described (7-9). For this, 100 µL of serial dilutions of the tailocin complex suspension in SM buffer were incubated with 900 µL of the target bacteria at 10⁸ CFU/mL and incubated for 40 min at 37 °C. As a negative control, 100 µL of SM buffer was used instead of the tailocin complex suspension. After this incubation time, serial dilutions of each condition were made before seeding 100 µL onto plates with LB agar medium, which were left overnight at 37 °C. To stablish the LKU the following formula was applied: $\frac{LKU}{mL} = - ln \left(\frac{Nk}{No}\right) ∗ No$

Where Nₖ represents the lowest dilution of the tailocin complex suspension that shows killing activity (the one that appears to have a reduced CFU number) and Nₒ represents the lowest dilution of the tailocin complex suspension that shows no killing activity, so that this value is similar to the negative control.

### COMBINATION OF PHAGE vB Pa-PAC2 AND TAILOCINS COMPLEX

### - Time killing curve assay.

A killing curve analysis was carried out in LB broth liquid medium with the isolates 1 and 4, which both were susceptible to the tailocins complex and the phage vB_Pa-PAC2 (10). For this purpose, an overnight culture of the *P. aeruginosa* isolates, were diluted 1:100 in LB and incubated at 37 °C at 180 rpm, until reaching a density of 10⁸ CFU/mL (OD₆₀₀ₙₘ=0.2). At this point, a 96-well flat-bottom plate was prepared with 200 µL of LB with 10⁵ colony formation units per mL (CFU/mL) from the overnight isolates culture, mixed with 10⁶ LKU/mL of the tailocins complex alone, 10⁶ PFU/mL of the seed of phage vB_Pa-PAC2, and the combination of both (referred to as Tailocins-PhagePA). A growth control was tested with the isolate alone and a negative control was conducted with LB broth medium alone. The plate was incubated with continuous orbital shaking at 540 rpm at 37°C for 24h and read at 600 nm wavelength every 15 min, using an EPOCH Microplate Reader (BioTek, Winooski, VT, USA).

### - Galleria mellonella model.

The *G. mellonella* model was adapted from previous versions described (10-12). Briefly, several groups of twelve *G. mellonella* larvae (n=12), obtained from a local pet shop (Harkito, Madrid), were infected by injection of 10 µL of 10⁴ CFU/mL, in the case of the isolate 1, and 10⁷ CFU/mL in the case of isolate 4 (both diluted in PBS), in the last left proleg, with a Hamilton syringe (Hamilton, Shanghai, China). The concentration of inoculum was selected after determination of the 50 % lethal dose (LD₅₀), i.e., the amount of inoculum that causes death of 50 % of the larvae 24 h post-infection. For preparation of the inoculum bacteria, 5 mL of LB broth medium was incubated overnight at 37°C with shaking at 180 rpm. The culture was then centrifuged (4000 x g for 15 min) and washed twice with PBS. One-hour post-infection, with isolate 1 or isolate 4, the larvae were injected in the right proleg with 10 µL with the treatments: the phage vB_Pa-PAC2 alone with 10⁷ PFUs/mL or with 10⁹ PFUs/mL, respectively, the tailocins complex alone with 10⁷ LKUs/mL or 10⁹ LKUs/mL, respectively, and finally, by Tailocins-PhagePA with the same concentration as used individually. As control infection group, the larvae were injected with PBS after one hour-post infection. In addition, to verify that the injection alone did not affect the larval state, a control was injected with PBS simulating the infection and treatment. Finally, toxicity control was performed to evaluate the toxicity of the treatment. The larvae were placed in Petri dishes and incubated in darkness at 37ºC. The number of dead larvae was recorded after 24 h and 48 h. The larvae were considered dead when they showed no movement in response to touch. The survival curves were plotted using GraphPad Prism v.6, and data were analysed using the Mantel-cox test.

### RESULTS

### - Identification TAILOCINS complex

The search for tailocins in the isolate 10 of *P. aeruginosa,* with bioinformatic tools, allowed us to find a PTLB cluster in one strain, as it was located between the *trpE* and *trpD* genes, as described in literature (13, 14). The nucleotide sequence of this cluster was analyzed for its classification into type and subtype. Currently, two types of tailocins have been described according to their morphology observed by transmission electronic microcopy (TEM): type R (rigid) and type F (flexible) (15). The annotation of the cluster sequence showed the typical structure of the tailocin cluster with a block of regulatory genes, the structural genes and the lytic genes. The structural genes were grouped into two blocks corresponding to a R-type and to a F-type tailocins, a tailocins structure that was previously reported in *P. aeruginosa* strains as PA01(5, 14).

The R-type tailocin from the complex was characterized as R1 subtype, as it showed 99.95% homology with the typing protein. The protein homology study of the F-type region resulted in the identification of a new F-subtype and therefore a new tailocin complex.

### - Host-range assay

The activity of this complex was tested by Spot test assay in 14 isolates of *P. aeruginosa* resulting in 9 being susceptible to the action of this complex, which corresponds to 64.29 % of the total (Figure 1). In previous studies, it was determined that the phage vB_Pa-PAC2 also infects 4 of these 9 strains susceptible to the tailocins complex.

It is now known that there are twenty different serotypes of *P. aeruginosa.* In the study collection, there were six different serotypes (O1, O3, O5, O6, O9 and O11). The tailocins complex was effective on five of them, the only one on which it did not act was O6. Moreover, none of the susceptible isolates presented the same serotype, which were: ST242, ST1072, STS285, ST198, ST312, ST360, ST1092, ST319 and ST701.

### - Time kill curve

The time killing curve assay performed with the seed of phage, vB_Pa-PAC2, showed that the action against infection of isolates 1 and 4 did not eradicate completely the growth of bacteria (OD₆₀₀ₙₘ: 0.716 and 0.0712, respectively). In the case of the tailocins complex it can be observed that it eliminates completely the infection (OD₆₀₀ₙₘ: 0.0030 and 0.0036, respectively). Finally, with the Tailocins-PhagePA it can be seen an improvement in the eradication of the bacterial infection (OD₆₀₀ₙₘ: 0.0011 and 0.0014, respectively (Figure 2).

### - G. mellonella infection model

The larval survival assay showed that the Tailocins-PhagePA was not toxic for the *G. mellonella.* In the case of the group of larvae infected with the isolate 1 and treated with the phage did not improve the survival at 24 h, since there is an 8.33 % of survival, same as the group of untreated larvae. On the other hand, treatment of this infection with the tailocins complex slightly improves survival by 33.33 %. A large increase (66.67 %) in survival of *G. mellonella* was observed in the group treated with the combination of both treatments (Tailocins-PhagePA) at 24 h (Figure 3A).

On the other hand, when the larvae were infected with the isolate 4 and treated with the phage alone or with the tailocins complex alone, it is observed a slight improvement in the survival (33.33 % and 41.67 %, respectively). However, it can be seen that the treatment with the Tailocins-PhagePA significantly increases the percentage of larvae survival up to 66.67 % (Figure 3B).

### SEQUENCE LISTING

FASTA tailocins complex (SEQ ID No 1)
>Phage tail-like bacteriocin (PTLB) or tailocin complex

ATAAGACGGCGCGAGGCGGAAGAAAAGAAGGCGGATGGCGTTTGCGTCATCCGCCTTTTTATGCCC
GGCTGGACCCACGCATTGGCTTCGCCCTACGCGGCACCGCTTTCGTAGGGCGAATATCGCGGCGCGA
TATCCGCCAGGGGGCAGGACGCCGAACCCGCCTCAAGGTGAAGTGATCGATATGTCCGGTTGGCGG
ATGACGCCTTCGGCATTACTTGCCCACGCAGAAGGCGCTTTCGTAGGGGGACATCACCACGCGATATC
CGCCAGGCGGAGCATGGTGTCGCTCCCTACGATGCTGGCAACGGCGGCCGCTCAGGATGCGATGCT
GTCAGCGCCCGGCTGTGCTCGACGGGCGCTTCCGGCTTGGTTCGCGCGCTGGTCGACGTAGGCGGCG
AGGTGATCGAGGGCCACGAACTGCATGGCCTTGTGGCTATCGTCGAGGGTCGTCACCGGCAGGGCG
ATCCGCCCGCATGCCAGGGCCTTGCTGAAGTTTTCCTTGTTCAGGTTGCGAAACCAGCGTTCGCGGAC
TTGCTCCAGCGGCACCAGGACGTCGCCGAAGATGCGGTAGACCAATTCCACGGTTTCCTGGCGTGGA
ATCGGAGTGCTAGGGGCGATGGATGGCTGCATGGTATTCCCTCCTGCGGCTACACGTCGTTGAAGGA
AATATAGCTCAGGTTGTTTTCTTGTTCAATAGCTGAAGTTGTAGAGCGGGCGAGCGCCAGGCGCTCAC
CGCACCAGGGACGGGCCGCGCCGGTGGCGTACGGTGGACCACCAGAAGACCCAACCGATCATGCTG
ATCTGGCGGCTGCGCATGTCCTCCGGCGAGTACTCCTCGTCCGGATATTCCTCGCGGTTGAAGCTGCG
CAGGCGAATGCCGCCGCCGGGCAGGCGATAGACGAACTTCACCCGCAGCATGCCGTCATGTTCGAGG
GCGTAGATCTCGCCATCGGTGATATGGGTGGTGGCGGTGTCCACGCCGATGGTGGAGCCATCCATGA
TCAGCGGTTCCATGCTGTTGCCGGTGAGTTGGGCGCAGATCGCCGCCGACGGATCGACGCCCGAGGC
ACGCAGCGTGGCATAGGAAAAACGCAGCTTGCGCCCCTCTATCTCGCGTACCGCAGTGCGTCCGGCG
CCGGCGGACATCTCCACTTCCTTGTACAGCGGCAGTTCCACTTCGTCTTCGTCCAGCGGTGTGTCGCTG
TCCCACGGATGCAGCGGTTCCAGCACCAATGGGCTGCCATCGGCCACGGACACGGCACTGCGCGCGG
CTGGAGCGCCTTCGCCGGTCTGCAGCCAGACCGGCGAGACGCCCAGGGCCGCCGCGATCTCGATCAG
CTTGCGGGTGCTCTGGGCCTTGCCCGAGGTCAGCTTGTGAATGGTGTTCTGCGAAACCCCTGCCGCTT
CGGCGAGGGTTTCCTGCTTCAGGTTGCGCATCGCCATGGCCTGCTTGAGGCGAGCGGCGAAGCTGTC
GGGCGGGATCTGGGTGCTCTTGTCCATGCTCGACAATCTACAGACCGATGGATTTTCTGTAAAGAGCC
TAGGTGTTGACGATAAATAGCTTTGGTTGTAATTTCTCTTCCGTCAGAAAGCGGAAGGGGTGAGTTCC
TCAACTTCCCACCCGCCTTTCAGAGATTTCACATGGGTGCAGGGACGCACCCGGTTGTCGGCGACCGA
CGATGCAGGTGCCGACTGCTTGGGTGCCGGGGCCATGGGAAAGCGCTCCGGCACCTGGAGGGCCGG
AGCGCCGTCCATGCAGACGCTCCGTCCCGCTCATTCATTCTGTCGCCGACCTGCTTCAGGTACGGCGGC
GTTGTACAGGGAGTCATTGCCATGTCGCGAGATACGCACTCGCCATTTCCGCGCAGAGGAACGCAGG
TCGCCGAACCGGCGCCGCGCATCTGTCATGTTCCATCCATTCGGCCGTCGGGCGTCGGGCACGCCAGG
GAGGCTTTCCATGGCTGACCTTGCCGATCACGCCAACGAACTGGTCCTGGCTCGCCTCGACGGCCTCC

TGGCGGCGCGCCCGGCGCTGGCCATCCGCGAGTCCGCGGAAGACTGCGAGGACTGCGGCGAGCCCA
TTCCCCAGGCGCGCCGCCGGGCGGCACCGGGCTGCAGTCGCTGCATCGACTGCCAGGACCGCCACGA
GCGCCGTTGAACCGACCTCATGCCGAGCCCTCGCGGGCAGTGAAAGGAGACACGACCGTGATCAAA
GCCATCGATGAAATGCTCAAGCTCTGGGCCGAGGAAATGCACGCGCCAGGCAGCAACGGAGGCGGC
TACGCCGGCGGCAACCTGATCGCCATGCTGATCGCCAGCAAGGGCGAGGTGGTCCGCGGCCACCGTG
GCAGCCGGGTGATCCTCGACCGTGTGGCGGAGGTCGATCGCCTGGTAAATCGCCTACCCGAGGAACT
GAAGAACGTGGTGGTGGAGCACTATCTCAATCGCGACAGCTTCCCCGAGCAGAAGTACCGCCACTGC
GGTTGCAGCCGCAACACCTTCTATCTACGCCTGCATGTGGCGCACCAGGGTATCCAGGACGGCCTGCT
GCGGCGGGTGGCCTGAGTTCCTGGACCGGATACCGTGTCCCCCCTGGAATTTTCCAACCGGCCTTCGA
GCCGGTTTTTTATGCCTGTCGCCCATGGAGGCGGCGAGCCCCATGCACAGCAGCGGACCCTGCACG
GTAGAGGCGGATTCGGGCTGGCGTAGCCATCCTTTTCGTCAGTCGATGCCGGGCTTTCCACATCGGAA
GTGGGTATGCGCTTGCCCGGATGCCTGGCGAAGGTTCCCAGGGCGGTATATCACCCTCATATCGTGG
TGCGTCTCCCTCCTATTGCAGTGGATTGTCGAGAAGGCATTGCCGGGCTGGGAATCGGGCGGTAAAA
AGTAGTCATTCTTGTAAAGGTGCGTCCCCAGGGAGGCACTCGTGAAGCACCGGAACCCGGCCCTGGC
GCCGGGTTTTTATTGCCCGGGAAAACGTTCGTGCGGACGTGCCCGGCGCTGCCTGCCAAGGAGGGA
CCCATGGGCAACGAACCGCAGACACTGACGGAAATGCCGCTCTGGGTACTGATCCTGCTCGCCGCGC
TGGGCGGCGTCAGCGGCGAGATGTGGCGTGCCGACAAGGCCGGTCTCGGCGGCTGGGCCTTGTTGC
GGCGCCTGGCGCTGCGCTCCGGCGCGTCGATTGTCTGCGGCGTGGCGGTGATGCTGCTGGCGTTGGC
TTGCGGCGCCGCGCTGCTGTTCGCCGCGGCGCTGGGCAGCCTGACCGCCGCGGCCGGCGCGGAGATC
GCCGTCGGTCTCTACGAACGCTGGGCCGCCCGGCGCCTGGGGGTTTGCGAGCTGCCCGAGGAACAGT
CGGACGACCGTGGCCCGCATTGAAACTGATCGAAGGAGTCAACCATGCCTGAACAGGCTGTTACGCT
CGAGGCTCTGTACGCGGCCATTGAGCAGGTACTGCGTGAGCGTCTGCCGGAGGCGCAGTTGATCGGC
TTCTGGCCAGGCGTGCCGGAAAATACCCCGGCGGTTTCCCTGGAAATAGCCGAGCTGCTGCCCGAGC
GCGATCCCGGTACCGGCGAGAGCGCCCTGCTGTGCCGCCTGCAGGCGCGGATAATGGTGCCGCCTGG
TGCCGATCGCCAGGCGGTATCCATTGCTTGCGGAATCGTTCGGACATTGCGCGAGCAGACCTGGAAC
CTGTCTCTGCAGCCGGCGCGCTTCGTACGCTCGGCCGTCGACGGCAGTCGCGAGGAGCTGAAGAGCC
TGCGTGTCTGGCTGGTCGAGTGGACGCAGTCGCTGCGTCTCGGAGACCCGGAGTGGGCCTGGGAGG
ACCAGCCGCCGGGCAGCCTGATGCTGGGCTTCGACCCGCAGACCGGGCCCGGCCATGAGCCGGACTA
CTTCGCTCCGGAGGCGTTGGCATGAGCTATGTCAGTGCGGAGCATGACCGCATGCTCGCCGCGATGA
TCCTGCCCTGCGTGGTGGTCGCCGTGGACCTGGCAGCGGCGCGGGTACGGGTGCGCTCCGGCGACTG
GACCAGCGGCTGGCTGCGCTGGCACTCCCTGGCAGCCGGCAAGGTTCGCCACTGGCGTGCGCCGAGC
ATAGGCGAACAGGGGATACTGCTCAGCCCGTCGGGCGGAGTGTCAATGGGCACCTTTATTCCCGGTC
TGTACGGCGATGCGGGCACGGCGCCGGACAACAGCGCCAGCAGTGAGACCTGGCGTTTCGACGACG
GCGCCTCGTTGAGTTACGACTGGGCTGCGCATCGCTACCGCGTCGAGCTGCCCAGCGGCACCGTGGA
AGTGAGGGTCGGCGCCAGCGAGGTGCGGGTCAGCGACGGGGCGGTCAGTCTCAAGGCGCCGAAGA

TCAGCCTGGAAGGACCGGTGGAGATCGCCGGGACACTGACGGTCAGCGGAGACATCCTCGGCGGCG
GCTCGATCATCGACACCGCCGGCAACAGCAACCACCACCCATTGAACATACGACGGGCTGCCGA
AGGGCGGTCCGTCGAACATTCAACCGGCCCGCGCGAGCGGGCCTTTTCGTTTGCGGAGTTCGCCATG
GGCAACACTCACAGCCATTCGGAGCCAGGCGCGGCCTGTCTCTGCCGGAGCGGGAGGGCAGGGCGA
TGATCGGGATGGATCGCCGTAGCGGGCTGCCCCTGTCCGGCCTGGCTCATCTTAAACAGTCCGTCGAG
GACATCCTGACCACCCCGTTGGGCAGCAGGCGCATGCGCCCCGAGTACGGCAGCAAGCTGCGGCGG
ATGGTCGACATGCCGGTGAGCGAAGGCTGGAAAAGCGCCGTGCAGGCCGAGGTAGCCCGTTCCCTG
GGGCGCTGGGAACCGCGCATCGCATTGTCTGCCGTGCGAGTCGTCGCGGTCGTCGATGGCCGCGTGG
ATCTGCTCCTGAGCGGCGTGTTCGAGGGCGAGAACATCAATATGGAGGTCTCGGCGTGATCATCGAT
CTTTCCCAGTTGCCGGAGCCAGAGGTTATCGAAAACCTCGATTTCGAGACGATTTACCAAGAGCTGTT
GGGCGACTTCCGCGAAGCCATGGCTGGCGAATGGACAGCGGAGGTGGAGTCCGATCCGGTTCTCAA
ACTTCTGCAACTGGCGGCCTATCGAGAACTGCTGCTGCGGGCGCGGATCAACGATGCGGCGCGGGCG
GTGATGCTGGCATACGCCAGCGGTGCCGATCTCGACCAGATCGGTGCCGGCTTCAATGTGCAGCGTT
TGCTGATCAGGCCCGCTCAGCCCGAGGTGGTACCGCCGGTGGAGGCGCAGTACGAGAGCGACAAGT
CGCTGCGCAATCGCATCCAGCTCGCGTTCGAGCAGCTATCCGTCGCAGGACCGCGGAACGCCTATATA
GCCCATGCGCTGGGCGCGGATGGAAGGGTGGCGGATGCCTCTGCGACCAGTCCGGCGCCCTGCGAA
GTGCTGATCAGCGTGCTCGGGGTGGAAGGCAACGGGCAGGCACCGGAAACGGTGTTGCAGGCAGT
GCGCCTGGCGCTGAACGCGGAGGACGTGCGTCCTGTCGCGGATCGGGTAACGGTGCGCTCGGCAGG
AATCGTTCCCTATCAGGTCAAGGCGCAGCTCTACCTGTTTCCCGGCCCCGAGGCCGAGCTGATCCGTG
CCGCCGCCGAGGCTTCGCTGCGCGACTACATTTCCGCCCAGCGCCGCCTGGCCGCGACATCCGGCGT
TCGGCCCTGTTCGCCACCCTGCATGTCGAAGGCGTGCAGCGCGTCGAACTGCAGGAGCCTGCGGCCG
ACGTGGTCCTGGATGAAACCCAGGCGGCCTATTGCACGGGGTACGCGATCACCTTGGGAGGCGTCGA
TGAGTAGCCGCCTGCTGCCGCCAAACAGGAGTTCTCTGGAACGCTCTCTGGGTGATGTATTGCCTGCC
GAGCTGCCGGTGCCGCTTCGTGAGCTTAACGATCCGGCACGCTGTGAGGCGGCCTTGCTGCCCTACCT
GGCCTGGACGCGCTCGGTGGACCGCTGGGACCCGGACTGGAGCGACGAGGCCAAGCGCAATGCGGT
AGCGACGTCCTTCGTCCTGCACCAGCGCAAAGGCACGCTGACCGCGTTGCGCCAAGTGGTCGAGCCG
ATCGGTGCGCTGAGCGAGGTCACCGAATGGTGGCAGCGAAGCCCGCTTGGCGTGCCGGGGACCTTC
GAGATCACCGTGGACGTCAGCGACCGTGGCATCGACGAAGGCACCGTACTGGAGCTGGAGCGCTTG
CTCGATGACGTCCGCCCGGTGAGCAGACACCTGACCCGGCTGGACCTGCGCATTACCCCGGTAATCCG
GTCCCGTCACGGACTGGCCGTGACCGACGGCGACACCCTGGAAATCTTCCCCTGGAAACAGTGACAT
GACGACCAATACTCCGAAATACGGTGGCCTGCTCACCGACATAGGTGCCGCTGCGCTGGCTGCGGCC
AGTGCAGCAGGCAAGAAATGGCAGCCGACTCATATGCTGATCGGCGATGCCGGCGGTGCGCCGGGC
GACACGCCGGATCCATTGCCTTCTGCGGCGCAGAAGAGCCTGATCAACCAACGCCATCGGGCTCAGC
TGAATCGGCTGTTCGTTTCCGACAAGAACGCCAATACCTTGGTTGCCGAGGTGGTGCTGCCAGTTGAG
GTAGGTGGCTTCTGGATCCGCGAGATCGGCCTGCAGGATGCCGACGGCAAGTTCGTCGCGGTATCCA

ACTGCCCGCCCAGCTACAAGGCTGCAATGGAAAGTGGCAGTGCGCGGACCCAGACCATTCGGGTGA
ACATCGCGCTCTCCGGCCTGGAGAATGTCCAGCTGCTGATCGACAACGGCATCATCTACGCCACTCAG
GACTGGGTGAAGGAAAAGGTCGCTGCCGATTTCAAGGGCCGCAAGATCCTGGCTGGCAATGGCTTG
GTCGGTGGGGGCGATCTTTCTGCCGACCGCAGCATTGGTCTGGCGCCTTCCGGCGTGACGGCGGGCA
GCTATCGTTCGGTCACGGTGAACGCCAACGGGGTGGTCACCCAGGGCAGCAATCCGACCACCCTGGC
CGGCTATGCGATCGGAGATGCCTATACCAAGGCCGATACCGATGGAAAACTGGCGCAGAAAGCGAA
CAAGGCCACCACCCTGGCCGGCTATGGCATTACCGATGCGCTGCGAGTCGATGGCAACGCCGTGTCA
TCCAGCAGGCTGGCCGCACCGCGTAGCCTGGCAGCCAGTGGCGATGCCTCCTGGTCGGTGACCTTCG
ACGGCAGTGCCAATGTTTCTGCGCCGCTGAGTCTTTCCGCTACCGGTGTGGCGGCGGGCAGCTATCCG
AAGGTGACCGTGGATACGAAGGGAAGGGTGACTGCTGGAATGGCGCTGGCGGCGACGGACATTCC
CGGGCTGGATGCTTCGAAGTTGGTCAGCGGGGTGCTGGCCGAGCAGCGTTTGCCGGTATTCGCGCGC
GGGTTGGCTACTGCTGTCTCGAACAGTAGCGATCCGAACACCGCGACCGTGCCGTTGATGCTGACCA
ATCATGCGAACGGACCTGTTGCCGGACGATACTTCTACATCCAGTCGATGTTCTATCCGGATCAGAAC
GGCAATGCTTCGCAGATTGCAACGAGCTACAACGCTACATCCGAGATGTATGTACGGGTGTCCTACG
CGGCCAACCCTAGCATCCGGGAGTGGTTGCCCTGGCAGCGTTGCGACATTGGAGGTTCCTTCACGAA
GACGACTGACGGATCCATTGGAAATGGCGTCAATATAAACAGCTTCGTCAATTCCGGATGGTGGTTG
CAATCGACATCGGAATGGGCGGCGGGTGGAGCTAACTATCCCGTGGGGCTGGCCGGTTTGCTGATTG
TCTACCGCGCACATGCAGACCATATCTATCAGACCTACGTAACACTCAACGGAAGCACATATTCGCGC
TGCTGCTATGCGGGCTCTTGGCGTCCGTGGCGGCAGAACTGGGACGATGGAAACTTCGATCCGGCCA
GCTACCTGCCAAAGGCGGGATTTACCTGGGCGGCTTTGCCGGGTAAGCCGGCAACTTTCCCGCCCTCA
GGGCATAACCACGATACCAGCCAGATCACCTCCGGCATCTTGCCTCTCGCTCGTGGTGGCCTTGGCGC
CAATACAGCGGCCGGAGCACGCAACAACATTGGTGCCGGAGTGCCGGCCACGGCGAGCCGGGCGCT
CAATGGTTGGTGGAAGGACAACGATACCGGTCTGATCGTCCAGTGGATGCAGGTGAACGTAGGAGA
TCATCCCGGTGGAATAATCGATCGTACCTTGACGTTCCCGATCGCGTTCCCTAGCGCCTGTCTGCATGT
CGTACCGACTGTCAAGGAGGTGGGGCGACCAGCGACGTCCGCGTCGACCGTTACGGTCGCCGATGTC
AGCGTCAGCAACACGGGATGTGTGATCGTTTCCTCCGAGTACTACGGACTGGCTCAAAACTATGGCA
TCAGAGTGATGGCCATCGGCTATTGAGGTGAAAATGATTTTTTTCCATGCCGCCACGGGCGGCTTTTA
TTCGAAGGAAATTCATGGGTCCCGCATGCCTCTGGAGGATGAAATGCATCCTCTCGAAGACGCCGAG
TACCAGGCTCTTCTTCGTGCGCAGAGCGAAGGGAAACGAATTGTCACGGACCACACCGGTCGCCCTA
TCTGCGTCGATCCACCGGCTCCGGCCAAGGACATTCTGGTCCAACGGAACGCATCTGGCGCGACCG
GCAGTTACAGCTCACCGACGGGCCTCTCGCTCGGCATCGTGACGAGCAGGACCTGGGAAAAACTACG
ACTCTGAGCCAAGAGCAGCTTCGTGAGCTAACTCTCTATCGCGCCGTTCTTCGCGACTGGCCTATTGCC
GCGGAGTTTCCCGACCTGAACGCAAGGCCCGAGCCGCCTGCCTGGCTCCAATCGCTCATCACCCCCTG
AACCCCGCCTTGTGCGGGGTTTTTCATTAATGGAGATCTACCTATGAGTTTCTTCCACGGCGTTACGGT
AACCAACGTCGATATCGGTGCGCGCACCATCGCGCTGCCGGCCAGCTCCGTCATCGGCCTCTGCGATG

TGTTCACGCCGGGGGCGCAGGCAAGCGCCAAGCCCAACGTTCCGGTGCTGCTCACCAGCAAGAAGG
ACGCCGCCGCGGCGTTCGGCATCGGCTCGTCTATCTACCTGGCCTGCGAGGCCATCTATAACCGTGCC
CAGGCCGTGATCGTGGCGGTGGGCGTGGAAGCCGCGGAGACTCCCGAGGCCCAGGCCAGCGCCGTC
ATAGGTGGTGTCAGCGCCGCCGGCGAGCGCACGGACTTCAGGCTCTGCTAGACGGCAAGAGCCGC
TTCAATGCCCAGCCACGTCTACTAGTTGCGCCGGGTCATTCGGCCCAGCAAGCGGTGGCCACCGCCAT
GGACGGGCTGGCCGAGAAACTGCGGGCCATCGCCATTCTCGATGGTCCCAATAGCACCGACGAGGC
GGCCGTCGCCTACGCCAAGAACTTCGGCAGCAAGCGCCTGTTCATGGTCGACCCGGGCGTGCAGGTT
TGGGACAGCGCCACCAATGCCGCACGCAAGGCCCCGGCTTCGGCCTACGCCGCCGGCCTGTTCGCCTG
GACCGACGCCGAGTACGGCTTCTGGTCCTCGCCGTCGAACAAGGAGATCAAGGGCATCACCGGCACC
AGCCGTCCGGTGGAGTTCCTCGACGGCGATGAGACCTGTCGCGCCAACCTGCTCAACAACGCCAATA
TCGCCACGATCATTCGCGACGATGGCTATCGCCTGTGGGGCAACCGCACCTTGTCCAGCGACAGCAA
GTGGGCTTTCGTCACCCGCGTTCGGACCATGGACCTGGTGATGGATGCGATCCTCGCCGGGCACAAG
TGGGCGGTGGACCGCGGCATCACCAAGACCTACGTGAAGGATGTCACCGAGGGCCTGCGCGCCTTCA
TGCGCGATCTGAAGAACCAGGGCGCGGTGATCAACTTCGAGGTCTATGCCGACCCGGACCTGAACAG
CGCCAGCCAGCTGGCCCAGGGCAAGGTGTACTGGAACATCCGCTTCACCGATGTGCCGCCTGCCGAG
AACCCCAATTTCCGTGTCGAGGTGACCGATCAGTGGCTCACCGAAGTTCTGGATGTCGCCTAAGGAG
CGCCCGTGATGATTCCGCAAACCCTGACCAATACCAACCTGTTCATCGACGGCGTGAGCTTCGCCGGT
GACGTGCCATCCCTGACCCTGCCCAAGCTGGCTGTGAAGACCGAGCAATACCGTGCCGGCGGCATGG
ATGCGCCGGTATCCATCGACATGGGCCTGGAGGCGATGGAGGCCAAGTTCTCCACCAACGGTGCCCG
CCGAGAAGCACTGAATTTCTTCGGCCTGGCCGACCAGAGCGCTTTCAATGGCGTGTTCCGCGGCTCCT
TCAAGGGCCAGAAGGGCGCCAGCGTGCCAGTGGTGGCTACCCTGCGCGGCCTGCTCAAGGAGGTCG
ACCCGGGCGACTGGAAAGCCGGCGAGAAAGCCGAGTTCAAGTACGCCGTTGCGGTCAGCTACTACA
AGCTGGAAGTCGATGGCCGCGAGGTCTACGAGATCGATCCGGTCAACGGTGTCCGCGCGATCAACG
GTGTCGACCAGTTGGCCGGCATGCGCAACGACCTCGGCCTGTAAGAGGAGCTCCGGACATGACCCAA
GAGAATCGACTGCCGGGCTGGCTGACGCTGGATGCTGACGCCGCCCTCGTTCGTCTCTCGCGTCCGGC
ACAGTGCAACGGGGTCAGCGTTGACACGCTGACCCTGCGTGCACCCACCGTGCGTGATATCCGCCTG
GCCGGCAAGGTGGCCGGCGACGATGCCGAGGAGCGCGAACTGCAACTGTTCGCCTCGCTGGCGCAG
GTCAGCCGCCAGGACCTGGAGGGGCTGAAGCTGAGCGACTACCAGCGGCTGCAAGGCGCCTACTTTC
GCCTGGTGCAAGACGACACGGACGACACCTTCGCGTATGCGTCAACTGGCGAGGCGCCTGGCCATTA
AGCTGGGCTTCCAGGCCAGCGAACTGGAGCGCATGACCCTGGGCGACCTGCTCTGGTGGCTCGCCGA
GGGCGAGGAGTGAGCGACGGCGCCGTCCGCCAGGCGAGCGGCGCCCGCGCTACTTCAGTAGGTCCG
CGGAGGCGGACATCGATAACAGACGAGACGAACCGTCATGAGTAAAGACATGGACCTGGTGGTCTC
CATCGGCGGCATCGCCGACCCCTCCTTGGGCAAGGCATTCGAGACCGTCAAGGCACGGCTCGACAGT
CTCCAGGAACGTTCCCGCCAGGCGTCCAGCCTGAGAGATGTGCTGGGAGACGCCATACGCCTGGAGC
GAGAACTCGCCGATATGCGCAAGGTCGGGGACCGCGGCGTTGCTGAGCATGCCCGGCAGCTTGGCG

AACGCCAGGAGCAACTGAAGCGACTCGGCATCGAGGCCAGGGCCGCGGGCGATGCCTACGCTCGAC
TGGGCGAGATGCAGCGTGGCCTGGATATGCAGGTCCGCGGCCTGCAACGGCTGGAGCAGGCCAGCC
AGGCAATGCCATTGGCTAGCGCATTTTCCGGACTGGTCGTGGAAGCCAGCAAGACGGCTGCCGGTTA
TCAAGCGCGGTTGCGCGACCTGGCGATCCGCAACGGCCTGGACGTCGGCCGGGAGCCAGCCTTGGCA
TCCCTGATCCAGGACAGTGCCAGCCAGAGCGGCCTGGGACGCACGGCGACGCTGGACATGCTGGAG
CACTTGAACGCCACCGGCATGGGGTTCGCCGCCGCAAATGAATCTGGGACTGGCGGGCCGCTTCG
GCTTTGGCCAAGGGATTGCTTCAGCCGAGGCTGCGGGGCTGGTTCGAGCGTTGCAACTGGCCCAGGG
TTCGGACTCGCCAGAGCAATTGTCCGCCTCCCTCGACCGCCTGGTCGTCCTGGGTAAAGGCAGAGTTG
GCAGTGAGGCCCTGGCGCGTCGCCTGCCTGCCTTGTTGTCAGCGCTGGGCAATGCGGGAGAGGCCAC
GGCCGGCGATGTCGGTGCACTGGGGGCCTTGCTGGAGATTCAGGCAAAGAACACCACGCCAGACAA
AGCGGACGTGCGGATGAAGGCCTGGCTGGAGTTCGTCGGCAGCGGCAGCTTGAAACGCGCTTATGG
CCAGGACTACGACCGGGACCTGGAAGCGCTGCGCAAGGACGGAGCGAGTCTGCTGGAGGCCAACCT
GGAGCTGGCTGCGCGCTATCGGGATAAGGGCGGCAAGCTCAGCGCCGGCGTGGCGTCGCCGGCGCT
TGAAGCCTATCGAGCGTCGCGCGGCGAGTTCCAGGGCTTGCTCGAATCCCAGCAGTCTTCCGCTGGCT
CTTCGGAGCGCGATGCGCGGCGTCGCGAGGGGATGTCCCAGGAGCTGTGGAAGGCTTCCAGCGACA
GTTGGGAGAGGGCGCAGACCGCTTTGGGCAGCGCCCTGAATCCATATCTGGACAACCTGGCCAAGG
GGAGCGCGGTACTCGGCGAGTCGACTGCGGAGCTGCTCGAAGCCTATCCGCGGACGACGGCCGGTC
TTACCGCCGCCGCAGGTGCGGTGTTATCCGGGTATCTCGCCTACAAGGGAGGGCGCGGCGCTATCGA
CGTGCTGCGTGGCGGTCGGCTCGGTCGGCGAGGGAGCGCTGCCGTCGGCGACCTGATCGAACGGGG
TGCAGGCCGGGTATCGGGTGGTAGTGAAATACAACGCGTGTTCGTTACCAACTGGCCGGTGCCGGG
AGGCGACTCTACGCTGGAGTCCGCGAGGAGGCCGGCACAACGAAAGCGCGGTCAAACACCACGCAG
GAAGAGAGGCAAGGGCGGTGGGGTGAAGGCCCGCTCCCTTCCATCCCTTGGCTTCTCGGCTGGAGG
AGGCTTGGGGGCGATGGCAGGAAAGCTGCCGCGCCTGTCGCGTTTGCCCATACGCAACGCGCCGCTG
CAAGTGGCCTCGTCGTTGATCGATGTTGCTGAGGTCTACTCCAGCGACCTGTCGGAGAGCGAAAAGA
CCATCGCCTATGGCGAGGCCGGTGGCTCCCTGGCTGGTTCCCTGGCTGGCGCCGCTCTGGGAGCAAG
CATCGGCTCGGTGGTGCCGGTGGTCGGTACGCTGATCGGTGGATTGGTTGGCGGCGCTATCGGCGCC
TGGGGCGGTAGCGAACTGGGGGGGCGTCTGGGACGCAGTCTGGCTGGCGATCCGCCGGCGGCCTCG
GACAACAAGCCGGCGGTGCCCGCACCACAGGCCGAGCCCGTCGCGGCTGCGCCCAACTGGACCTTCG
CGCCGCAGATCAACCTGACGGTGCAAGGCAACGTACACGAGCCGCAGCGCCTGGCCGACGAGTTGCT
GCCCTACCTGCAACGCATGCTTGTCGACTTCGCCGACGAGCGGCAGCGGCGCAGCCTCTACGACCCGG
CGATGGTCTAAGGAGTCCCCATGGCATATCTGGAACAATTGCAGGCCGGCCTGAGGTACCTGGGTCG
CGCCGGCGAGTCCGGACGCAAGAGTCTGGACAAGGTGGTCGCTCCGGTGAACGGTGCGATCAGCGA
GATCCGCGGCGCAGCCGCGGAGCTGGAGAACCTGCCCGGCGTATCGCCGGAAATGGCTGCCCGGCT
GCAGCGTGCCATGCGCGGCATCGGCCAGGCGCAGGGCAAGGTGAACCGCGTGGTCTCCACCTATGA
CCGGGCGAGCCGGGCGTTGCTTGGTATCGACGAACGCCTGGATGCGCTGAAGGTGCAGGTGAACAG

TGCCGCGCAGGCGGTCGGCAAGGTCGCTGGCGACATCAGTCCGACGCTGGCGGGGGTGCTGCCGTC
GTGGCTGCTGGCACCCTCGGCGACGCCCCGAGCGAGGCCGCAGCGCCCTTGCCGCACCTGCTGGTA
CTGCAGCCGCTGACCGCCAATGCCCAACCGTTCTACTTCAACCTGAATACTGCCGCCTTCGACGCCCTG
CAGCGCAACAGTGCCTACAACTGGAGCGGGCAGGTGCGCCTGGGTCGGCGGCCGGCGCTGCAGAGC
GTCGGCATGGGCGAGGAGAGCATCCTGCTCAAGGGGGCGGTATTCCCGCTGCGTCGACAGGTAGGT
AACCAGGAAAAGGTCGTCGGTCTGGAGCAGCTCGAAGCGCTGCGCCGGTTGGCGGAGCGGCGTGAG
CCGCTGATCCTGAGCAGCGGCTACGGCGAGGTGCAGATGGGCCTCTGGTGTCTGGTGCGGATCAGCG
AGAACCAGAGCGCCCTGCTGGGCAACGGCGCTCCCCGCAAACAAACCTTCGACCTGGAGTTCAAGCG
CTATGGCGACGACCTGCCGAACCGCTGACGGCGACATGCTGGACAGCCTCTGCTACCACGTCTATGGC
CATCTGTTGGGCTGCGTCGAGGCGACCCTCGACGCCAATCCCGGGCTGGCCGATGAGCAGCAGCCAT
TCCGCGCCGGCTTGCTGATCAGTTTCCCTGACATGCCGGTGGTCAATGTCGAACAGGTGCGCCTGTGG
GATTGATCGACCGCTCACCCGCAACCCCGCCTTGGCGGGGTTTTCTTTTCTGGAGAAACCAGGTGCA
ACCGAGTTTCCGTATCGTTGCCGACGGCACCGACGTCACCCAGCGGCTGAATGACCGCCTGCTCAAGC
TGACCCTGCTGGACAAGCCGGGCATGGAGTCCGACAGCCTGACCTTGAGGATCGACGATCGCGATGG
ACAGGTGGCCTTACCCAGGCGCGGTGCGGTGCTGGAGGTTCATCTCGGCTATGCCGGCGAGCCACTG
ATGCGCATGGGACGCTTTACCGTGGACACCTTGCAGTGGGCTGGTCCGCCGGACTGCCTGACCGTCAC
TGCCAAGGCCGGCGACATGCGCGGCAGTGGCAAGACGATACGCAGCGGAGGTTGGGAGGGCACTA
CCCTGGCTCAGGTCTGCCGCGATGTTGGCGCACGCAACGGCTGGCGCGTGGAGTGTCCGTTGCAGGT
GGCGATCGCCCGGGTCGACCAGGTCAACGAGTCCGACTACCACTTCGTCACCCGTCTGGCGCGCCAAT
ACGACTGCACCGCCAAGCTGGCCGAGGGCATGCTCATGGTGCTGCCGCGACAGAGCGGGCAGAGCG
CAACGGGGCGTCGGATCGAACCTTTGGTGTTGGGACGTGCTGACGTCGGCAGCTTCGACGTTACCTA
CGACGACCGCAGCCTGATGAGAACGGTGAAGACCCGCTACCAATTGCCCGGCAGCGGCGAGGTCAA
GAGCGTCGAGCTGAAGAACCCGAAGGCACCGGCTACGGCTACGGGCGAGCATGTCGACCGGCATCT
CTATGCCAGCCGTGGAGAGGCCGAGCAGGCGGCGAAGGCTCGCCTGGCGAGCTTCAGCCGCTCCAG
TGCCAGTGTGCGCCTGGAGCTGCCAGGGCGTGGCGACCTGTTCGCCGAGCGCAGCCTGCTGCTCCAA
GGCTTCAAGGCGGGAATCGACGGCGAGTTCCTGATCGACTCGGTGGAGCACACCTACAGCTCCAGCG
GATGGACCACTGTCGTGCAATGCAACGGCGGCCGAGGCGGCAAGGGGTGACGCCTGAAACCCATCG
GAGTGCAGGAGGATCGATGAAACTGACCGAGCAGCAATTGCTGCGCATTTTTCCCAACGCCCGCCTC
GTCGCGGGCGTTTTCGTTGCGGCGTTGCAACGGGCCATGGACGAGAGGGAGATCGACACACCGGCG
CGGCGTACCGCGTTTCTCGCCCAGGTCGGCCACGAAAGCAGCCAGTTGACCCGGCTGGTGGAGAACC
TCAATTACAGCGCCCAAGGCTTGGCGGCGACCTGGCCGAGCCGCTATCTCGGCCCCGACGGGCAGCC
CAACGCGTTGGCCTTGCGGCTGGCGCGCAATCCGCAGGCGATTGCCGACAACACCTACGCCACGCGC
AACGGCAATGGCGACGAAGCGTCCGGCGATGGCTGGCGCTTTCGCGGGCGTGGCTTGCTACAAATCA
CCGGGCGTGCCAACTACCGGTTGGTCGGCGAGACCCTCGGCGAGCCGCTGGAAGCCGAGCCCTGGC
GCCTGGAGCAGCCCGTGCCGGCGGCCCGCAGCGCCGCCTGGTGGTGGGCCGGTCACGGGCTCAACG

AGTTGGCCGACCGCGGCGAGTTCGCTGCCATCACCCGCCGCATCAACGGCGGCCTGAATGGCCAGGC
GGAGCGCCTGGCGTTGTGGCAGCGGGCCAGGGCGGTGCTGTCATGAGCCGGCTCGCTCTGCTCCTGC
CGGCCGTGTTGCTGGTCCTGCTGGCCGGCGCCTTGCTCGGCGGCGGCCTGGTTGCCCGCCATTATCGT
CCGCAACTGGAGGAGGCCCTGGGCCAACTCACTGCCAGCCGCGTCGCCAGCGGCCAGCTCGAGGCTT
TGCTCGATGAGCAGCAGCGCGCTGGCGGTGCGGGCGAGCGCCGAGAGGCGCGCAAGGAC
GTCGAGCAGGCACTCGGCGAGGCCAGGGCGCAGGCCGCGGAGCAGTATGCCGCGGCCGTGCGTCTG
CTCCAGGAACCCGACATTGGCACGGACTGCCAGGCGGCAGGTGCGGCGATCGACCGGGAGCTGGGA
CTATGACTCGTCTCCTGCTGGGGCTTTGTCTGCTTTTCGCGGGCTGCGCAGCCTCACCGACGACACCTC
GCCCCGTGCGCGTCGAGGTTCCCCTGGCAGTGCCCTGCCGTGTACCTGACGTGCGCCCGCCGAGCTGG
GCCGGCGCCACGCTGAAGGCCGGCGATTCGCTGCAGGCCAAGGTTCGCGCATTGCTCGCCGAGCGCC
GCCAGCGGCAGGGCTACGAACTCGAATTGCAGGCGGCATTGCGTGCCTGCCGCTGAGACACTGGGC
GCATATGGACGTCGATGGACGTGTGTCCGGCGCATATTGCCGTGTATTGATTCCGCAGCGTTGTCGA
GCCGGGAATCGGTCGGTACAACGTAGTCATGCTTGTACAGGTGTGTCCCCCCAGGGATGTCACCTGC
AACCTCAGAGCCCGGCCAGTGTGCCGGGCTTTTTCGTTTGCATCCGACAACGGCTCGGGACGTGGAG
GCTCCTCGCCGACCGCGTACCGCGCCACGGCCGACCGTCCTGGGGACGGGGCCAGACGACCCGCCTG
GTGCGGGTCTTTTCATGTGCATAAAGGAGAGTTTTCCATGTCCATCCTGACTCAAGGTACCCAGATCT
ACGCCCTGGTTCCGCCGGTATCCGGTACCGGTGCCGCTACCGTCCTGGAGATCGAAGGCGTGACTTCG
TTCAACCCGGGCGGCAATCCGGCCGACCAGATCGAAGACCCGTGCCTGAGCGACACTTCGCGCAAGT
ACAAGAAAGGCCTGCGCACCCCTGGCCAGGCGACCCTCGGCATCAACGCCGATCCACGCCTGGCCAG
TCACGTTCGGTTGTTCCAACTGTCCGAGAAAGATGGCGAGACGAGCGTCAAGTGGGCCATCGGCTGG
TCCGACGGAATCGACGTAAAGCCGACCGTCAGCACCGAAGGCGACGATTTCGTGCTGCCGCCGGCGC
GCACCTGGTTCACCTTCGAAGGCTACGTCAGCGACTTCCCCTTCGATTTCGCCAGCAACACGCTGGTCG
CTACCCAGGCCACCATCCAGCGTTCCGGCGCCGGCAAGTGGACGCCGAAGTCGGCTTGAGGAGCGCG
TAGATGAATCTCAACGAACTTCGTGCGGCGGGCGGTTTCATCGAGTCGGCGCTGGTGCGCAAGGAGA
TTACCTGGACCCGCGTTCCCGCCGGCAGGAAGAAGGCGGTCAGCGACACCTTCCAGGTGTTCGTCCG
ACGCAACAGTTTTGGCGCGGTGGAGCGCCTGTTTTCCGCCGAAGGCGACCAGCAGAGCCGCAACGCG
CGCTACCTCGCCGAATGCATTCGCCTGGGCGAGACGGAGAGGAAAGCCTGACCTACGAACAGGCCT
ACGACCTCGACCCGGCGCTGGGCTTCCTGCTGCTGCAGGCGGTCGGTGAGGTCAACCGGGTCGAGGA
CGCGGAAAAAAACTGACCCCCGCCGACGAGGTTTGGCACGAACTGGTGCTGAACGGCGTCGGCGGG
TCGACCATTGCCGAGGCCAAGGAGCGGCTCAGCTACGCCGAGTACCGCGCCTGGGTCGCCTATCTGA
ACAAGCGCGGCTCGCTCCATCCGGGACACCGGCTGGAGCTTGCGCTGGCTCGGATCGCTGCGTTGCTC
GGGCATGCGCTGGGAGCGGACGCCGATCCCGACGCGTTCCGTCCGCATATGGCTCTCCAGCCCCTTTC
GCTGCACCAGGCGATGGATCAATGGGCATGACAGGCCACCCCGCTGCGGCGGGGTCTTTTTCTGGAA
GACATGAATCATGGCCACGAATACCGACGGCAGCCTGACGCTCGACCTGGTCCTTCGCAGCGAAGGG
TACAGGGCCGGGATGGACAAGGTTGGCAGGATCAACGATCAGAAAATGCGCGCCATGGAGGCGCG

CGCGGAAAAGGCTGGCAAGGCTATCGGCAAAAGCCTGGACAGTTCGGCACTGATTGCCAGCAGCGT
GCTGGACCAGGCGCTGGACATGCTGGGCAGGACCAGTCGCCAGGCGGGTCAGGCCAAGAAGCCTGT
GCAGAGCGCCCAGGACAAGGTACTGGCCGAGTGGAAGACCCGGCAGAAGGAGCTGGGCGAAGCCT
GGAAGAGCTATCGCGAACCACTCCAGGATCTGTCCAAGCTCAACGAAGCACTACTGAAGAACTCTTC
CGACAAGCTCGACAAGGCGCTGCTCAATCTCAGCGAGACCGGCAAGCTGTCGCTTGCCAACGTGGGC
AAGGCCGCCTACGCCGATGCCGCGCCTCGCCTCGCGGCAGATGACCCTGATGCTGCTGGACGGGC
TGTTTGGCTGGGTCGCCAGCGTCGGTACCGAGAAGCCCAAGGTCGACGACAAGGCGGCAAGGGAC
AGGCGAAGGCCGGCGACGACGAGAAGGAACAGCCGTCGCTCCAGTCGCAGGTCTTCAAACAGTGGC
TGTTGCAGATGAACAGTGTCTGGGGCGCCTACCGCGCCGCTGCAGGATATCTCCGGGATGACCGA
CGAGCTGTTCAGGAATGCGTCGGAGAAGCTCGAGAAGTCGCTGTTCAATTTCGCCACTACCGGAAAG
CTGTCCTTGAGCAACTTCGCCAAGACGGTGATCGATGATGTCGCCCGGATCGCCGCGCGGCAGCTTTC
AATGCTCGCCCTGGACGGATTGTTCGGCTGGATGAATGGCAAGGCCGGCATCACCGAGGCGCAACTG
GCCAGCCAGAAGCCCTATACCTCGCTACTGGAAAAGGCCCGCGCAGCTGCGGGACAAGCGGCAGCG
GGCGCTCCCGCGGCCCTGGGTGCCGCGCCAATGCCGGCAGCGGCGATGGATGTCGGCGCGATGGTG
GCCACTGCTTCCGGGCAGACCGGGGACGGTTCCAAGGTATCGGGTGGAGGGGCCTCGGCGAGCGCT
GGCAAGCCGGTGGGCAGTTGGGTCGAACAGATGGACGCCTCCTGGGCGAGCCTGCGCGACCAGGCG
CAGGACGTCTCGGGAATGATGGACATGCTGTTTACCAACGCCTTCACCAATATGGAGAACGCCCTGTT
CACCTTTGCCACCACGGGCAAGCTGTCGTTCAAGGATTTCGCCGACTCGGTGATCCAGGATATGGCGC
GGATCGCCGCGCGGCAGGCGACGCTGCAGATCATCGGCGGCATCGTCGGTGCGGTCAGCGGGTTCTT
CGGTAGCGGCGCAACGGCGGGCTCGCGGATTTCCGACTACACCGGCTCGGACATGGCCAATTGGGTC
AGCAAGCAACGCGCCGGAGGCATGCCTGGGTTCGCCAGGGGCGGTGCCTTCAACGATGGCATCCAG
AGCGCGCCGGCGCTGTTCAGCATGGCCGGCGGTCGTCCGGCGCTGATCGGCGAGCGTGGCCGGAA
GCCATCATGCCGCTGAGTCGCGGTTCCGATGGCGTGCTCGGCGTGCGCGCGCTCGGCGGCGGCGAGG
GGGGGGGGCAACGTCTTCAATTTCTCCACCAGCGTCAGCCTGGGCGGCGGCCGCGAGGGCGCGGCG
ACGGCCAGCGGCGACGACGGTACGGGACAGCAGCTGGCGGGAATGATCAACGATGCCGCGCGCAA
CGTGGTGGCGCAGGAGCTGCGCCCCGGCGGCCTGGTATGGAGGATGGTGAATGGCTGATCTGGAAC
GCTTTACCTGGGACATCTCGATCGATTCCGCCGGCCAGGCGAACCAACTGGTACGCCAGGTGCAGTA
CGGCGGCGGCTACAGCCAGGCGCTCGGTGACGGGCTGAACAACCTCAGCGAGACCTGGCAGGTTTC
GCGTACCGGCGATCTCGCGCTGATCGGCCCGATCCGCGATTTCCTCAAGCGCCACGGCGGCTACCGCT
CGTTCCTCTGGACCTTGCCCACTGGCGAACCGGTACGGGTGCGCGCCCAGGGCTGGCAATTGCGGCC
GCGCGGCAACGGTGTGTTCACCCTGAACACCACCTTCCAGCAAGTCTTCAATCCGTGAGGTAAGCATG
ACCATCACAGCCGATGACCAGGCCCTCGAGCCTGGGGCGCTGGTGCGCCTGTTCGACCTGGATTGCA
CCGGGTTCGGCGGCGAGATGCTGCGCTTCCACGGCCACCTGCAGCAGGGGCCGATCCACTGGCAGGG
CAACGCCTACCAAGCTTGGCCGCTGGAGGCGCGCGGCTTCGAGCAGCGCGGCGACGGCCGGGCCAG
TTCGCCGACCCTTAGCGTGGGCAACATCGACGGCAGCATCAGCGCGCTCTGCCTGTTCTTCGATGGCC

TGGTAGGCGCGCCTGACCGTGCGCGAGACCTATGCGCACTACCTGGATGCGGCCAACTTCGCCGA
AGGCAACCCGCAGGCCGACCCCTCCCAGGAGCGCCTGAACATCTGGTTCCTCGAGCAGAAGACCGCC
GAGAACAGCGTCCAGGTGACCTGGGAGCTGTCCGCTCCGCCGGACTTCCAGGGCCAGCAGATCCCGG
CGCGCCAGATCACCTCGCTGTGCCACTGGTGCATCACCAACGAGTACCGCGGGCAGGACTGCAACTA
CACCGGCACGGCGATGTTCGATGCCGACGGCAATCCGGTGGACGATCCGGCGCTGGACCGCTGCGGC
GGCCGGGTCAGCGATTGCAAGCTGCGCTTCGGCGCGGACAACCCGCTGTCCCACGGCGGCTTCGCCG
GCGCCGGCCTGGTCAGGATGTGAGCATGGAACTGAGCCGCAGCTTGCAGCGGGCCATCGCCGCACA
CGCCGCCCGCGAGCATCCGCGCGAATGCTGCGGGCTGATCGTTCGCGGTGCGCGTCAACGCCGCTAC
GTGGCCTGTCGCAACGCAGCCGGATCGCCCAGCGAGCACTTCGTGATCGATCACCAGGACTGGTGCG
CCGCCGAGGACCAGGGCGAGGTCCTGGCCATCGTCCACAGCCACCCGGATGTTCCGGCCACGCCGAG
CATGGCCGATCGGGTCAGTTGCGAACTGCATGGTCTGCCTTGGGTGATCCTGTCCTGGCCGGAAGGC
GATGTCGCGCATCTAGCGCCGGAGGGCTATCGGGCGCCGCTGCTCGGCCGCGAGTTCGCCCACGGCG
TGCTCGACTGCTGGAGCCTCTGTCGCGACTGGTACCGCCGCGAGGCAGGTCTGGAGCTTCCGGACTAT
CCGCGCCGCGACGGTTGGTGGGAAACCGGCGAGAGCCTCTACGAGCAGCACTATGCGGCGGCTGGA
TTCCGGCCGGTGCCGCTGGCCGGAATCCGCCGCGGCGACATGCTGGTGATGCAGGTCGGGAGGGCG
CTGCACCCGAACCACGCGGGCATCTACCTGGGCAATGACTGGCGTCTGGACAGCGAGCCGGTCCAGG
CGCTCGGCGGCGACGGACCGTTCCTGCTGCACCACCTGTACGGACGGCTGTCGACCCGAGACGTGTTC
GGCGGACCCTGGATCGAACGCACGCGCCTGGTCTTGCGGCACACGCGGATGCCGCAGTGAACGACAT
ATTCAAGCGAGCCGTCGGAATCGGCTCTTCACGAGAGGAACAGGTCCATGAGTGACACCCTGAGTCA
GGGCCTCACCACCATCCGTCTGTACGGGGTTCTGGGCAAGCGCTTCGGCCGCATGCACGGCCGATTGT
TGGAAAGCGGCACGGTACGCGAGGCGATGAGTGCCTTGAAGCACACCATGGAGGGATTCGAGACGT
TCATGCGCGAGGCGGAATCGAAAGGGCTGACCTTCGCCGTGTTCCGTGGGCGTACCAACCTGTCCGG
CGAGCAACTGGACATGCGCGGACGCGAGGATATCCGCATCGTGCCGTTGGTGATCGGGAGCAAGCA
GGCGGGGCTTTTCCAGACGATTCTGGGGGCTGCCTTGATTGTCGTGGGTGGATTCACGACGTTCTTCT
CCGGTGGAACCAGTTCGTTCCTGGTAACCGCTGGAGTCAGCATGTTGGCGGGCGGCGTCATGCAGAT
GCTCAGCCCCCAACCCAAGGGCCTGAAGGGCCGAGAGGCCCCCGAGAACGCCCCCAGCTATGCCTTC
GGCGGCCCGGTCAACACCATCGCCCAGGGCCATCCGGTCGGCGTGCTCTACGGCAAGCGCCGCATCG
GCGGCGCGGTGATCAGCGCCGGCATCTATGCCGAGGACCGGCTGTAGCCGGCAACGCCGTAACAGG
CCCGCCATGCGCGGGCGTTTTTTTGCCTGAAGGAACGTCATGAACAAGACCATCACGGCCACAAGG
GTGGCAGCAAGAAGCCGCGCCAGCCGGTGGAGATGCCGGACTCGGTGCGCTCGATCGCGCGGGCGA
AGATTCTCCTGGCACTGGGCGAAGGCGAGTTCGACGGTGGCGTCGACGGCCGTTCGATCTACCTGGA
CGATACGCCGCTGCTGGCGGCGGACGGCTCGGTGAACTTCCCCGGAGTGACCTGGGAGTTCCGTCCG
GGCTCGGTGGACCAGGAACACATTGCCGGTGTGCCCGCCGTGGAAAACGAACTGGCGGTCGGTGTC
GAACTCAAGAGTGACGCGCCCTGGGTCCGCGCGGTGAACAACACCCAGCTCTCGGCGGTGCGCCTGC
GCCTGTCCTGGCCGGCCATCCAGCGCCAGCAGGAAAACGGTGACGTGGTCGGCTACCGCATCGACTA

CGCGATCGACATCGCCGTCGACGGCGGTGCCTGGCAGGAAGCGCTGAAGGCTTCGCTGGACGACAA
GTCCACCAGCCGCTACGAGCGCTCCCACCGTGTCGACCTGCCGGAGGCGGGGAGCGGCTGGCAGGT
GCGCGTGCGCCGCCTGACGCCGAACCAGAACAACAACCGCGTCGCCGACACCATGCGGGTGGAGGC
GATCACCGAGGTGATCGACGCCAAGCTGCGCTACCCGAACACCGCGCTGCTGTTCGTCGAGTTCGAT
GCCAGCCAGTTCCAGAGCATTCCGCAGATATCGGTGGAAGCGCGGCCGGCGGGTGCGGGTGCCG
AGCAACTACGATCCGCAGACCCGTAGCTACAGCGGCACCTGGGACGGCTCGTTCAAGTCGGCCTGGA
CCAGCAACCCGGCCTGGCACTGGTACGACATCGTGTTGCACAAGCGCTTCGGCCTCGGTCGGCGGAT
CGACGCGAGCATGGTCGACAAGTGGTCGCTGTACCGCATCGCCCAGTACTGCGACCAGTCGGTGCCC
GACGGCAAGGGCGGCCAGGAGCCGCGCTTCAGCTGCAACCTGTACCTGCAGAGTCGCGCCGAAGCC
TGGACAGTGCTGCGCGACCTGGCAGCGATCTTCCGCGGCATGTCCTACTGGTCCGGCGCGGAAATGG
TGGCGGTATCCGACATGCCGGAGGACGAGGCCTACACCTTCTCGCCGTCGAACACCGTGCGTGGCGA
CGACGGCAGCCACTTCAACTACAGCAGCAGCCGCCAGCGCGATCGCCACACCCTGGCCCTGGTCAACT
ACGACAATCCGGGCAACGGCTACCAGAGCCAGCCGGTAGCGGTGAACAATGATCGCGCGCAGCGCC
GCTACGGCATCAGCCAGTTGGAGATCACCGCGATCGGCTGCACCTCCGAGGGCGAGGCGCAGCGGC
GTGGCCAGTGGGCGCTGCTGACCGAGGAGCTGGAGCAGGACGCGGTGACCTTCCGCACCGGCATGG
ATGGCCGTGGGCTGGCGCCGGGGAAGATCATCGCCGTAGCCGACCCGGTCAAGTCCGGCAAGCAGA
TCGGCGGACGCCTGAGCGCGGTGGATGGCCGCGCGCTGACCCTCGACCGCGACGTCGAGGCCCGACC
CGGCGATCGCCTGCTGGTCAACCTGCCGAACGGCAAGGCCGAGGCGCGCAGCGTCCAGTCGGTGGT
AGGCCGCGTGCTGAGCGTGACCGCCGCCTATTCGGAGACGCCTCGGCCCCAGGGGCAGTGGGCGCT
GCAGAGCAACAGCCTGACCACCCAGCGCTTCCGCATCATGAGCATCACCCGGCCGGAGGACAATCTT
TTCGAGATCACCGCGCTGCAACACAACGCGAGCAAGTTCGACGCCATCGACAACGGTGCGCGCATCG
AGCTGCCGCCGGTCACCAGCATTCCGCCGGGCGTGCAGGCGCCGCCGCAGAACGTGCGGATCAAGG
CTTTCACCAAGGTCGACCAGGGGTTGGCGGTGACCAGCCTGTCGGCCTCCTGGGATGCCGCGCCGAA
CGCGGTGGCCTACGAGGCCGAATGGCGCAAGGACTCGGGCAACTGGGTGCGGGTGCCGCGAACCTC
GGCGCTCGGTTTCGACGTGCCGGGCATCTATGCCGGTCGCTACCTGGTGCGGGTACGCGCCTTGAAC
GTGATGGAGGTCGGTTCGGTCTACGCCAGCAGTGTGGAAACCGCTCTCGAGGGCAAGACCACACCGC
CGCCGGCGCTGGCCTACCTACGCTGCGTGGCCGGCCCCTGGCGCATCGGCCTGGAGTGGGGGTTTCC
GGCCAGCGGCGCGGCGGACACCGCTTACACCGAGATCCAGCAGTCCGCCACGCCCGGCGGCAGCGA
GGAGACCGCACGGGCGCTGGGCCTGTTCGCCTACCCAGGCAATACCCACCTGGTATCGCCGATACCG
GCCGGCGAACGGCTGGCGTTCCGCGGTCGCTTGATCGACCGTAGCGGCAACGTCGGCGCCTGGTCGA
ACTGGGTCACCGGCACCAGCTCCAGCGACGCCAGCGAATACAACCAGTTGATCACCCAGGAGTACGT
CGAGTCGGCGCTGGGCCAGCAGTTCTTCTCCGATATCGAGCGGATGCAGGTGGATATCGGGGGCTTG
CAGAAGCAGGTCGGCGACCTCGCCGACGTTCTGCTGTACGACCCGGCCAAGGTCTACGCGAAGAACG
ACATGGTGCGACAGGGGCAGCGGTTGTACCAGGCACTGAAGGCTGTGCCGGCGAAGACGGCGCCGC
CGAACGCGGCCTACTGGTCCGATATCGGCCAGTCGCTGGAGACCGCCAACGGGCTGGCGCAGCAGG

TGGCGAGCCATACCGCTGAAATCAGCGAACTCGACGGCAGGATCGAAGCAGCGGCATCGAATCTGG
ATGTGCTGCAAGCTGCCGCCCGCGGGAACCGGCGACCGGAGAAGGCGGATGCGCTGAAGGGC
TGGGACACCATTGCTCGAGCTGCCACCGAAGTCACCGTGCGGGCGAACGAGGACGAAGTGCAGGCG
AAGCGGACGAGCTTGCTTGAGGCGTGGACCGGGACGGCGGAGGGCAGGATCGCCACCGTCGAGTC
GGTCGTTGCGTCGAACAATGCCGTAACCGTCCAACGCCTGGATCAGCTATCCGGACAGGTTGCGAGC
AATGCCTCGGCCATCAGCACCGAACAGACCGTCCGCGCCAACGCGGACAGCGCCCTGGACAGCGG
GTGGATACCGTCAGCGCACGCACCGATACCAACGAGGCGAACATCCAGACCACATCTCAAGCGGTTA
CCTCGCTGGATGGCAACGTCAAGGCGCTCTACAGCGTGAAGCTCCAGGCGCATGCCAACGGGCAGAA
GTACGCCGCTGGCTGGCAACTGGGCTTCGACAGCGGTACGAGCGTGACGACCATGGCGTTCCAGGCT
GATCGGTTCCTCTGGTTCAACAGTTCCAGCGGGCAGACAGTGGCGCCGGTTTCGATTGTCGGCGGCC
AGATGTTTATCAACAACGCGATGATCCAGGATGGGTCAATCACCAATGCGAAGATCGGCAACGTGAT
CCAGTCGACCGCCCTCGGTGCCAACGGCGAGCCGCTGTGGAAGTTGGATAAGGGCGGCGCGTTCACA
ATGAACAGCGCCACGTCTGGCGGCTTCATGAGGCAGACAGCGGAGGCCACCAAGGTCTACGACGCG
AATCTTGTGCTGCGGGTACAGATCGGGAATCTAGACGTATGAGCTACGGAATCCGCCTGAGAAATGC
GGCCGGCTCCATCCTGATGGAGCTCACCGGCCAATCGGCGCGCACGGTCTACCGGCAGTCGCTCGGC
GCCATCACCAACGGGATGACGGTGACGGTGCCGGGTTTTGATCCTGCGCGCGGTGTTGTGTTCATCAT
CGCGAGCGGAAACGAATTCGGTGAAGTGCCCCGATACACAATTTCCGGAAGCGTGGTGACGTTCCAC
TGGAACGGTTCATCCGGAACAACTTATGTACTGCATGCGGTGATGTTCTCATGAGCTACGGAGTATTA
ATTCGCGGGGATGCTGGCAAACAATAATCGACGATAGTAATCCGTGCATTCATTTCGCTGCGTCGG
GAACTTATGGACATACGACCGGCAGAGAAACTGTTATTCAATATGCCTTTCCAATACAGTCCCCGTAT
GAGCCGTATGTCTTCGTGCGCCCAAATGGTCCGCATCAAATCTATTTGTTCAGGCATATCGGCGCCCC
GGGGAACTGGACTGGATTTGCATTCTGGCAGACGATCTATCGGGACGTGGACCCTCCAATCTACGGC
GGAAAGTGGAAAGCTGGCGCGGTCATGTTGCCGAAAACCGGTGGGTGGGGAATGCAGGTTTTCGAC
TCCCAGTCGCGTGTGATGTTCGACAGTAACCGGGACATCGTTCGCTATGTCGGTGGCGCACAGGTTTG
GAATAAGTATTCGTACAACCCGAGCTGGCCAGGCGGGATGGCACTACAAACGTGGTATCTGCCGTTC
ACATATGGAGTTGAGGCCTACTTCCAAGTCAGCCATTTCAATGTCAAAGCATTCATAACGTTAGAAGC
GCCGCGCATAGGTTTTCTTGAGAACTCAATGAGCTTGATATTTGTTTCATCAGTTGTAGAGTTTGAAA
CTAACCATCAGTTCAATTGGCCGCTTATTGTAGTGGCGTAAATATATCTGGAGGACTATATGGCTTGG
TATTCCACAGGCACGGTTGCTGTCACGCTGAATTCGCCGACAGTCACCGGCACTGGGACCGCATTTTC
CGCCAACGCCCGGGTCGGCGATGCATTTCGCGGACCCGATGGGCGTTGGTACGAGGTCACAAACGTC
GCCAGTTCGACGGTCATCTCGATCAAACCCAACTACCAGGGCAGCACGGCCAGCGGCCAGCCCTATG
CAGTGGCGCCGATCCTGGGCTACGACAAGGACCTATCAGATCGTTTCAACCAGATCGCGATGGACTG
GGGGGCGACCCTTGCGGGCATAAAGCCGTGGGCCCTGTCCAATACCGGCACGCAAGCGCAGGCGGA
CATGGGAATGACGGCGGTGGGGCGGGGACTCAACGCCGCAGCGACTGCTGAGAATGCCCTGAGCTT
TATTGGTGGCATGCCGAAGTCGATGTCCAATCTGCGGGCTGTCAGCGACGCCAATAATGTCCCGAAC

GAATGTGGGTTCTACGGTATCGGAGCGTCGCCTTGGGCGAACTTGCCGCCGGGGGTCGACGGTATCA
ACCCTATCGGATCCATGCTCTACCACCATCCGTACGATGTGAGCACCGCCGTGCAGATGCTCATTCCGC
GAACCTCTGACCTCATGTACTTCCGCCGGAAGCTCTCCGGCAACTGGAGCGCATGGGTGCGGCTACTC
TCGGATAAGCAGCTTGTAGGTACGGTATCTGTTGACGGGTCGAATGTTCCGAACGGTGCGGTCATGC
AGCAGAACGGGACCACGGCTATCAACGTGGGCACCAGTCTGCGTTTCGCCGACGGCACTCAGATTAT
CTACGCGAAGCTCCGTCTGGAATTCAGCGCGGTAGACATCTTGACCCGCCAGTACACGTTCCCCATGA
GCTTTTTCGAACCCCCGAATGTCACCGCTACTTTAATTCAAGGTCAGCAGTCGGATATCAATCCATTGC
AGTTCCAGCAACTCGGTCCGGTATTGGTCGCTGCTACTACTGTTAGCGCGTGCAACGTACGAGTCATG
CGCCCTACTTACGTATCGAGTGGCTGGGCTTCTGGGAACTTCATCGACTGTTCTGTCAACGCGGTAGG
GAGATGGCGCTAATGAAGTTCTTGCTAAAACCCGACCTGCAAGTCGGATTGCCTGGCCAAGAACGGG
TAAGCTCGGTTTCTGTAAACGGATTGCGGTTGACTATCGACGGCGTAGAGTTCGATTTTTCTCCACTTG
CAGTGGGTGGGTACTTGCCTCCGGAGGCATACATCAACATAACCCCCCTGCAAGAGGTGGAGGTTCG
AAGCGACTTTCTTCTCGTACGCTACATCCACCAAGTGACAGCAGACATTCTGACTGCTTATCGCGCCGA
GATTGAACCAATTTTGATGGAAGTTGACGGACCTGTGGAGCTACCGAAATGAACATTGACTGGAGCC
AACTGAGAACCCCCGAGCAGCAGGCCGCCGAACGCTTGCAGGCTGAGTACGATGCCGCAGCCGCGG
CGCGGGCAAATGCCTACCGCCTGGAGAGTGACCCGCTCAAGACCGAGGCTGAATTCGATGCGATCAA
GGCCGGCACCGAGCCGGACTACTCTGACTGGATCGCCAAGGTAGAAGAGATCAAGGGGCGATACCC
CACTTCCTCGAACCCGCCCGTCATGTAAGCAGGCTTCAGCGCCACCCCGAAGCCTGTCTTGACAGTAG
CATCCCCTGCGCCGGCCGCTCCGGCCGGCGCTCTTCCCGACATCCCGCTGTTGCACCCATCCTCCCCACT
GGGCTATGTTCCCCAACATGGCTCCGTGAGACCGCTATCGTGATCCACCTCAGCAGCATCATCACCTCC
CTCATCAAGGCATTCGCCCGTTGGCGCTGGCGCGCCTGACTTCTTCTTCGCCGGCCTGGCCGGTCACGT
CTCCCTGCGTCCCCCGTTTTCCACAGGCATAATGCCGACGTTCCACTCTCCCTTCCTCCGCGAGCGCGCC
GGGAAACGGGTGTGGCGAAAGCCGGGTGATGCGATTCGCATAAGAATGAAATCAAGAGGTTACAG
CCAGCATGC

### REFERENCES

1. Thrane SW, Taylor VL, Lund O, Lam JS, Jelsbak L. Application of Whole-Genome Sequencing Data for O-Specific Antigen Analysis and In Silico Serotyping of Pseudomonas aeruginosa Isolates. J Clin Microbiol. 2016;54(7):1782-8.
2. Potter SC, Luciani A, Eddy SR, Park Y, Lopez R, Finn RD. HMMER web server: 2018 update. Nucleic Acids Res. 2018;46(W1):W200-W4.
3. Altschul SF, Gish W, Miller W, Myers EW, Lipman DJ. Basic local alignment search tool. J Mol Biol. 1990;215(3):403-10.
4. Saha S, Ojobor CD, Li ASC, Mackinnon E, North Ol, Bondy-Denomy J, et al. F-Type Pyocins Are Diverse Noncontractile Phage Tail-Like Weapons for Killing. J Bacteriol. 2023;205(6):e0002923.
5. Blasco L, de Aledo MG, Ortiz-Cartagena C, Blériot I, Pacios O, López M, et al. Study of 32 new phage tail-like bacteriocins (pyocins) from a clinical collection of Pseudomonas aeruginosa and of their potential use as typing markers and antimicrobial agents. Sci Rep. 2023;13(1):117.
6. Kutter E. Phage host range and efficiency of plating. Methods Mol Biol. 2009;501:141-9.
7. Kageyama m. studies of a pyocin. I. Physical and chemical properties. J Biochem. 1964;55:49-53.
8. Baltrus DA, Clark M, Hockett KL, Mollico M, Smith C, Weaver S. Prophylactic Application of Tailocins Prevents Infection by. Phytopathology. 2022;112(3):561-6.
9. Williams SR, Gebhart D, Martin DW, Scholl D. Retargeting R-type pyocins to generate novel bactericidal protein complexes. Appl Environ Microbiol. 2008;74(12):3868-76.
10.Bleriot I, Blasco L, Fernández-Grela P, Fernández-García L, Armán L, Ibarguren C, et al. Studies in vitro and in vivo of phage therapy medical products (PTMPs) targeting clinical strains of K pneumoniae belonging to the group ST512. Antimicrob Agents Chemother. 2025:e0193524.
11.Peleg AY, Jara S, Monga D, Eliopoulos GM, Moellering RC, Mylonakis E. Galleria mellonella as a model system to study Acinetobacter baumannii pathogenesis and therapeutics. Antimicrob Agents Chemother. 2009;53(6):2605-9.
12.Blasco L, Tomás M. Use of Galleria mellonella as an Animal Model for Studying the Antimicrobial Activity of Bacteriophages with Potential Use in Phage Therapy. Methods Mol Biol. 2024;2734:171-80.
13.Scholl D. Phage Tail-Like Bacteriocins. Annu Rev Virol. 2017;4(1):453-67.
14.lbarguren C, Bleriot I, Blasco L, Fernández-Garcia L, Ortiz-Cartagena C, Arman L, et al. The world of phage tail-like bacteriocins: State of the art and biotechnological perspectives. Microbiol Res. 2025;295:128121.
15.Ghequire MG, De Mot R. Ribosomally encoded antibacterial proteins and peptides from Pseudomonas. FEMS Microbiol Rev. 2014;38(4):523-68.

## Claims

1. A composition comprising at least one bacteriophage, or a nucleic acid encoding therefor, and at least one Phage tail-like bacteriocin (PTLBs), or a tailocin or tailocin complex, or a nucleic acid encoding for the same, and, optionally, a pharmaceutically or veterinary acceptable excipient; wherein the at least one bacteriophage and the at least one Phage tail-like bacteriocin (PTLB) or a tailocin or tailocin complex, are both specific against the same particular bacterial target species, or at least against bacterial strains within the same particular target species.

2. The composition according to claim 1, wherein the composition comprises at least two or more bacteriophages, or nucleic acids encoding for the same, having lytic activity against one or more target species, wherein the at least one Phage tail-like bacteriocin (PTLB) or a tailocin or tailocin complex, is specific against the same particular target species of at least one of the bacteriophages present in the composition, or at least against bacterial strains within the same particular target species.

3. The composition of any one of claims 1 to 2, wherein the target species is a *P. aeruginosa* strain.

4. The composition of any one of claims 1 to 2, wherein the target species is an antibiotic-resistant *P.aeruginosa* strains.

5. The composition according to any one of claims 3 or 4, wherein the bacteriophage specific against *P. aeruginosa* is *Pseudomonas* phage vB_Pa-PAC2 (NCBI:txid3127731), and the Phage tail-like bacteriocin (PTLB) or a tailocin or tailocin complex, is specific against P. aeruginosa is the tailocin complex of SEQ ID NO 1 or any functional variant thereof.

6. The composition of any one of claims 1 to 5, which is a liquid, semi-liquid, solid or lyophilized formulation.

7. The composition according to any one of claims 1 to 6, which comprises a concentration between 10⁴ and 10 ¹² PFU/mL of each bacteriophage.

8. A composition as defined in any one of the preceding claims, for use in the treatment of an infection in a mammal.

9. The composition for use according to claim 8, for use in the treatment of an infection of the respiratory tract.

10. A composition as defined in any one of claims 1 to 7, for use for improving the condition of a mammal by modifying the microbial flora in said mammal.

11. *In vitro* use of a composition as defined in any one of claims 1 to 7, for decontaminating a material.

12. A composition comprising at least one Phage tail-like bacteriocin (PTLB) or a tailocin or tailocin complex, for use in a method of treatment of an infection of a target species in a subject in need thereof, wherein the at least one Phage tail-like bacteriocin (PTLB) or a tailocin or tailocin complex, is administered to the subject prior to, simultaneously or subsequently to a composition comprising at least one bacteriophage; and wherein the at least one bacteriophage and the at least one Phage tail-like bacteriocin (PTLB) or a tailocin or tailocin complex, are both specific against the same particular target species, or at least against bacterial strains within the same particular target species.

13. The composition for use according to claim 12, wherein the composition comprising at least one Phage tail-like bacteriocin (PTLB) or a tailocin or tailocin complex, and the composition comprising at least one bacteriophage can be jointly present as a single composition or as separate compositions.

14. The composition for use according to any one of claims 12 or 13, wherein the target species is selected from any one of the bacterial pathogens selected from the list consisting of: *Pseudomonas aeruginosa, Escherichia coli, Klebsiella pneumoniae, Acinetobacter baumannii, Salmonella enterica, Shigella flexneri, Enterobacter cloacae, Proteus mirabilis, Staphylococcus aureus, Staphylococcus epidermidis, Streptococcus pneumoniae, Streptococcus pyogenes, Streptococcus agalactiae, Clostridium difficile, Enterococcus faecalis, Enterococcus faecium, Bacillus cereus, Listeria monocytogenes, Vibrio cholerae, Vibrio parahaemolyticus, Campylobacter jejuni, Helicobacter pylori, Burkholderia cepacia, Burkholderia pseudomallei, Neisseria meningitidis, Neisseria gonorrhoeae, Haemophilus influenzae, Yersinia enterocolitica, and Yersinia pestis.*

15. The composition for use according to any one of claims 12 to 14, wherein at least the one bacteriophage is *Pseudomonas* phage vB_Pa-PAC2 (NCBI:txid3127731) and the at least one Phage tail-like bacteriocin (PTLB) or a tailocin or tailocin complex, is the tailocin complex of SEQ ID NO 1 or any functional variant thereof, and wherein the target species is *P. aeruginosa*
